(19) **Europäisches Patentamt**
**European Patent Office**
**Office européen des brevets**

(11) **EP 1 615 870 B1**

(12) **EUROPÄISCHE PATENTSCHRIFT**

(45) Veröffentlichungstag und Bekanntmachung des Hinweises auf die Patenterteilung:
**25.06.2008 Patentblatt 2008/26**

(21) Anmeldenummer: **04726111.0**

(22) Anmeldetag: **07.04.2004**

(51) Int Cl.:
***C07C 57/05*** (2006.01)

(86) Internationale Anmeldenummer:
**PCT/EP2004/003690**

(87) Internationale Veröffentlichungsnummer:
**WO 2004/089856 (21.10.2004 Gazette 2004/43)**

(54) **VERFAHREN DER HETEROGEN KATALYSIERTEN PARTIELLEN DIREKTEN OXIDATION VON PROPAN UND/ODER ISO-BUTAN ZU ACRYLSÄURE UND/ODER METHACRYLSÄURE**

METHOD FOR THE HETEROGENEOUSLY CATALYZED PARTIAL DIRECT OXIDATION OF PROPANE AND/OR ISOBUTANE

PROCEDE D'OXYDATION DIRECTE PARTIELLE CATALYSEE HETEROGENE DE PROPANE ET/OU D'ISO-BUTANE

(84) Benannte Vertragsstaaten:
**AT BE BG CH CY CZ DE DK EE ES FI FR GB GR HU IE IT LI LU MC NL PL PT RO SE SI SK TR**

(30) Priorität: **09.04.2003 US 461136 P**
          **09.04.2003 DE 10316465**

(43) Veröffentlichungstag der Anmeldung:
**18.01.2006 Patentblatt 2006/03**

(73) Patentinhaber: **BASF SE**
**67056 Ludwigshafen (DE)**

(72) Erfinder:
 • **MACHHAMMER, Otto**
 **68163 Mannheim (DE)**

 • **CRONE, Sven**
 **67117 Linburgerhof (DE)**
 • **BORGMEIER, Frieder**
 **68163 Mannheim (DE)**
 • **MÜLLER-ENGEL, Klaus, Joachim**
 **76297 Stutensee (DE)**
 • **ADAMI, Christoph**
 **69469 Weinheim (DE)**
 • **DIEFENBACHER, Armin**
 **76726 Germersheim (DE)**

(56) Entgegenhaltungen:
**WO-A-99/20590         DE-A- 19 924 532**

EP 1 615 870 B1

**Beschreibung**

Vorliegende Erfindung betrifft ein Verfahren der heterogen katalysierten partiellen direkten Oxidation von Propan und/oder iso-Butan zu wenigstens einem der Zielprodukte Acrylsäure, Methacrylsäure, bei dem man einer Reaktionsstufe, die abgesehen von einem Eingang für das Reaktionsgasausgangsgemisch, gegebenenfalls weiteren Eingängen für Hilfsgase, und einem Ausgang für das Produktgasgemisch gasseitig abgeschlossen ist, ein Propan und/oder iso-Butan, molekularen Sauerstoff und wenigstens ein inertes Verdünnungsgas enthaltendes Reaktionsausgangsgemisch mit einem Eingangsdruck $P^1$ zuführt, in der Reaktionsstufe durch Überleiten des Reaktionsgasausgangsgemischs bei erhöhter Temperatur über einen im festen Aggregatzustand befindlichen Katalysator das im Reaktionsgasausgangsgemisch enthaltene Propan und/oder iso-Butan direkt partiell zu wenigstens einem Zielprodukt oxidiert und/oder ammoxidiert, und das Reaktionsgasgemisch als wenigstens ein Zielprodukt enthaltendes Produktgasgemisch mit dem Ausgangsdruck $P^2$ aus der Reaktionsstufe heraus und mit diesem Druck $P^2$ in eine Aufarbeitungsstufe führt, die gasseitig, abgesehen von einem Eingang für das Produktgasgemisch, gegebenenfalls weiteren Eingängen für Hilfsgase, und einem Ausgang für das Restproduktgasgemisch, abgeschlossen ist, in der Aufarbeitungsstufe aus dem Produktgasgemisch der Reaktionsstufe darin enthaltenes Zielprodukt in eine Flüssigphase grundabgetrennt und das dabei verbleibende, nicht nur Propan und/oder iso-Butan sowie gegebenenfalls Propen und/oder iso-Buten enthaltende, Restproduktgasgemisch mit dem Ausgangsdruck $P^3$, für den $P^3 < P^1$ gilt, aus der Aufarbeitungsstufe herausführt und im Restproduktgasgemisch enthaltenes Propan und/oder iso-Butan in die Reaktionsstufe zurückführt.

Acrylsäure und Methacrylsäure sind wichtige Zwischenprodukte, z.B. für die Herstellung von Polymerisaten.

Ihre Herstellung durch heterogen katalysierte partielle direkte Oxidation von Propan und/oder iso-Butan in einer Reaktionsstufe ist bekannt (vgl. z.B. EP-A 529853, EP-A 603836, EP-A 608838, EP-A 895809, DE-A 19835247, DE-A 10051419, DE-A 10122027, EP-A 1254707, EP-A 1254709, EP-A 1192987, EPA 1090684, DE-A 10254279 und in diesen Schriften zitierte Literatur).

Als Oxidationsmittel wird normalerweise molekularer Sauerstoff verwendet, der zum Beispiel in reiner Form oder im Gemisch mit sich bezüglich der partiellen Oxidation im wesentlichen inert verhaltenden Gasen (z.B. $N_2$ in Luft) dem Reaktionsgasausgangsgemisch zugesetzt werden kann. Inerte Verdünnungsgase wie $N_2$, $H_2O$, CO, $CO_2$, He und/oder Ar etc. nehmen die Reaktionswärme auf und halten das Reaktionsgasgemisch außerhalb des Explosionsbereichs. Generell werden in dieser Schrift unter inerten Verdünnungsgasen solche Gase verstanden, die sich beim einmaligen Durchgang des Reaktionsgasgemisches durch die partielle Oxidation zu weniger als 5 mol-%, bevorzugt zu weniger als 3 mol-% und besonders bevorzugt zu weniger als 2 mol-% umsetzen. Als Katalysatoren werden in der Regel im festen Aggregatzustand befindliche Multielementoxide eingesetzt. Die Durchführung der Reaktionsstufe ist an den im festen Aggregatzustand befindlichen Multielementoxiden sowohl im Katalysatorfestbett, - wirbelbett oder -bewegtbett (-fließbett) möglich.

Der Arbeitsdruck in der Reaktionsstufe kann gemäß der Lehre des Standes der Technik prinzipiell sowohl unterhalb von Normaldruck (= 1 bar) als auch oberhalb von 1 bar liegen (vgl. z.B. DE-A 19835247, DE-A-19924532, EP-A 895809 und DE-A 10261186). In der Regel liegt er zum Zweck der Überwindung der Strömungswiderstände in der Reaktionsstufe leicht oberhalb von Normaldruck.

Nachteilig an der heterogen katalysierten partiellen direkten Oxidation von Propan und/oder iso-Butan zu wenigstens einem der Zielprodukte Acrylsäure, Methacrylsäure ist die vergleichsweise ausgeprägte Reaktionsträgheit des Propans und iso-Butans. Sie ist ursächlich dafür, dass bei einmaligem Durchgang des Reaktionsgasgemisches durch die entsprechende Reaktionsstufe selbst bei erhöhten Temperaturen im Regelfall nur ein Teilumsatz des Propans und/oder iso-Butans erzielt wird.

Eine Zielsetzung der heterogen katalysierten partiellen direkten Oxidation von Propan und/oder iso-Butan zu wenigstens einem der Zielprodukte Acrylsäure, Methacrylsäure besteht daher darin, bei möglichst niederen Temperaturen bei einmaligem Durchgang des Reaktionsgasgemisches durch die Reaktionsstufe einen möglichst hohen Umsatz des Propans und/oder iso-Butans bei gleichzeitig möglichst hoher Selektivität der Zielproduktbildung, d.h., eine möglichst hohe Raum-Zeit-Ausbeute an Zielprodukt bei möglichst geringem Energieaufwand, zu erzielen.

Ein weiteres Erfordernis für eine wirtschaftliche Durchführung einer heterogen katalysierten partiellen direkten Oxidation von Propan und/oder iso-Butan zu wenigstens einem der gewünschten Zielprodukte besteht ferner darin, im Produktgasgemisch enthaltenes nicht umgesetztes Propan und/oder iso-Butan weitgehend in die Reaktionsstufe zurückzuführen. Im Stand der Technik werden dazu die folgenden Vorschläge gemacht.

Die DE-A 10119933 empfiehlt bei der Herstellung von Acrylsäure durch heterogen katalysierte partielle direkte Oxidation von Propan aus dem Produktgasgemisch die in selbigem enthaltene Acrylsäure entweder durch Absorption in ein flüssiges Absorptionsmittel grundabzutrennen und das dabei resultierende flüssige Gemisch aus Absorbens und Acrylsäure anschließend in an sich bekannter Weise rektifikativ, extraktiv und/oder kristallisativ bis zur Reinacrylsäure aufzuarbeiten oder die Grundabtrennung der Acrylsäure aus dem Produktgasgemisch in eine Flüssigphase durch fraktionierende Kondensation vorzunehmen, wie es z.B. in der DE-A 19924532 beschrieben ist und das dabei resultierende wässrige Acrylsäurekondensat z.B. durch fraktionierte Kristallisation weiterzureinigen.

Hinsichtlich des bei solcher Grundabtrennung der Acrylsäure in eine Flüssigphase hinein verbleibende, nicht umgesetztes Propan enthaltenden, Restproduktgasgemisches empfiehlt die DE-A 10119933, das Propan aus dem Restproduktgasgemisch abzutrennen und das abgetrennte Propan in die partielle direkte Oxidation zu Acrylsäure rückzuführen. Als Abtrennmethode werden dazu z.B. die fraktionierte Druckrektifikation oder die Extraktion mit einem hydrophoben organischen Lösungsmittel (das das Propan bevorzugt zu absorbieren vermag) und nachfolgende Desorption und/oder Strippung mit Luft zum Zweck der Freisetzung des Propans empfohlen.

In völliger Entsprechung empfiehlt auch die EP-A 1193240 bei der partiellen direkten Oxidation von Alkanen wie Propan das im Restproduktgasgemisch enthaltene nicht umgesetzte Alkan (ebenso wie in der vorstehenden Schrift des Standes der Technik bevorzugt gemeinsam mit als Nebenprodukt gebildetem Alken) von selbigem abzutrennen (z.B. absorptiv oder adsorptiv) und in die partielle Oxidation rückzuführen.

Nachteilig an der Rückführweise von nicht umgesetztem Alkan und gegebenenfalls als Nebenprodukt gebildetem Alken in die Reaktionsstufe gemäß den Empfehlungen des Standes der Technik ist jedoch, dass die Abtrennung des Alkans und gegebenenfalls Alkens aus dem Restproduktgasgemisch, in welchem das nicht umgesetzte Alkan normalerweise in vergleichsweise hoher Verdünnung enthalten ist, vergleichsweise aufwendig und mit besonders hohen Druckverlusten verknüpft ist. Letzteres weist von einer Anwendung erhöhter Drucke in der Reaktionsstufe weg, müsste bei der Rückführung des abgetrennten Propans doch immer auf diese rückverdichtet werden.

Nachteilig ist daran ferner, dass im Restproduktgasgemisch sonst noch enthaltene, für die heterogen katalysierte partielle direkte Oxidation sich vorteilhaft auswirkende Bestandteile (z.B. Wasserdampf, der in der Regel Aktivität und Selektivität der katalytischen Aktivmasse fördert, oder Restsauerstoff, der nicht hochverdichtet werden muß) nicht in die Reaktionsstufe rückgeführt (sondern ausgelassen) werden und selbiger bei Bedarf immer wieder frisch zugeführt werden müssen.

Mit ursächlich für den Vorschlag, im Restproduktgasgemisch enthaltenen nicht umgesetzten Kohlenwasserstoff separat, d.h., abgetrennt in die Reaktionsstufe rückzuführen, dürfte u.a. die Überlegung sein, die im Kreis zu führende Gasmenge (und mit ihr auch die Menge an Reaktionsgasausgangsgemisch) möglichst gering zu halten, um auf diese Weise die diesbezüglich aufzuwendende Förder- und Verdichterleistung (das im Kreis geführte Gas muss auf den Eingangsdruck des Reaktionsgasgemisches vor Eingang in die Reaktionsstufe rückverdichtet werden, da es auf dem Weg durch die Reaktionsstufe, die Aufarbeitungsstufe und die Abtrennung aus dem Restproduktgasgemisch einen Druckverlust erleidet, welcher zur Überwindung der Förderwiderstände verbraucht wird und wieder zugeführt werden muss) sowie die erforderlichen Volumina zu minimieren. Eine weitere Zielsetzung dürfte dabei sein, die Einsatzstoffverluste minimal zu halten.

Die Aufgabe der vorliegenden Erfindung bestand daher darin, ein Verfahren der heterogen katalysierten partiellen direkten Oxidation von Propan und/oder iso-Butan zu wenigstens einem der Zielprodukte Acrylsäure, Methacrylsäure zur Verfügung zu stellen, bei dem die aufzuwendende Verdichterleistung und die Einsatzstoffverluste auf andere, vorteilhaftere Art und Weise minimiert werden und gleichzeitig die Raum-Zeit-Ausbeute bei minimiertem Energieaufwand optimiert wird.

Demgemäss wurde ein Verfahren der heterogen katalysierten partiellen direkten Oxidation von Propan und/ oder iso-Butan zu wenigstens einem der Zielprodukte Acrylsäure, Methacrylsäure bei dem man einer Reaktionsstufe, die abgesehen von einem Eingang für das Reaktionsgasausgangsgemisch, gegebenenfalls weiteren Eingängen für Hilfsgase, und einem Ausgang für das Produktgasgemisch gasseitig abgeschlossen ist, ein Propan und/oder iso-Butan, molekularen Sauerstoff und wenigstens ein inertes Verdünnungsgas enthaltendes Reaktionsgasausgangsgemisch mit einem Eingangsdruck $P^1$ zuführt, in der Reaktionsstufe durch Überleiten des Reaktionsgasausgangsgemischs bei erhöhter Temperatur über einen im festen Aggregatzustand befindlichen Katalysator das im Reaktionsgasausgangsgemisch enthaltene Propan und/oder iso-Butan direkt partiell zu wenigstens einem Zielprodukt oxidiert, und das Reaktionsgasgemisch als wenigstens ein Zielprodukt enthaltendes Produktgasgemisch mit dem Ausgangsdruck $P^2$ aus der Reaktionsstufe heraus und mit diesem Druck $P^2$ in eine Aufarbeitungsstufe führt, die gasseitig, abgesehen von einem Eingang für das Produktgasgemisch, gegebenenfalls weiteren Eingängen für Hilfsgase, und einem Ausgang für das Restproduktgasgemisch abgeschlossen ist, in der Aufarbeitungsstufe aus dem Produktgasgemisch der Reaktionsstufe darin enthaltenes Zielprodukt in eine Flüssigphase grundabtrennt und das dabei verbleibende, nicht nur Propan und/ oder iso-Butan sowie gegebenenfalls Propen und/oder iso-Buten enthaltende, Restproduktgasgemisch mit dem Ausgangsdruck $P^3$, für den $P^3 < P^1$ gilt, aus der Aufarbeitungsstufe herausführt und im Restproduktgasgemisch enthaltenes Propan und/oder iso-Butan in die Reaktionsstufe zurückführt, gefunden, das dadurch gekennzeichnet ist, dass $P^1$ so gewählt wird, dass $P^3 \geq 1,5$ bar beträgt und man das Restproduktgasgemisch in zwei Teilmengen gleicher Zusammensetzung aufteilt, davon die eine Teilmenge als Auslass ausschleust und die andere Teilmenge als Kreisgas rückführt und als Bestandteil des Reaktionsausgangsgemischs auf den Eingangsdruck $P^1$ verdichtet wieder der Reaktionsstufe zuführt. Damit unterscheidet sich das erfindungsgemäße Verfahren u.a. dadurch von der in der EP-A 495504 offenbarten Verfahrensweise, dass sie in und/oder nach der Aufarbeitungsstufe vorab des Auslass keine katalytische Kohlenmonoxidoxidation aufweist. Auch bedarf es beim erfindungsgemäßen Verfahren keiner Kohlendioxidwäsche des Restproduktgasemisches.

Entsteht in der Reaktionsstufe als Nebenprodukt Propen und/oder iso-Buten, so bleiben diese Verbindungen über die Aufarbeitungsstufe normalerweise mit dem Propan und/oder iso-Butan vergesellschaftet und werden gemeinsam im Kreisgas befindlich in die Reaktionsstufe zurückgeführt.

Alle Druckangaben in dieser Schrift verstehen sich, soweit nichts anderes explizit erwähnt ist, als Absolutdrucke.

Normalerweise enthält das Restproduktgasgemisch wenigstens 2 Vol.-%, oder wenigstens 5 Vol.-%, meist wenigstens 10 Vol.-%, an von Propan und/oder iso-Butan sowie von Propen und/oder iso-Buten sowie gegebenenfalls von Sauerstoff verschiedenen (z.B. CO, $CO_2$, $H_2O$ und/oder $N_2$ etc.) Bestandteilen (in der Regel handelt es sich dabei um im Produktgasgemisch enthaltene, leichter als das Zielprodukt (bei Normaldruck) siedende Bestandteile).

Während die beim erfindungsgemäßen Verfahren partiell zu oxidierende organische Vorläuferverbindung (d.h., das Propan und/oder iso-Butan) in der Praxis häufig flüssig gelagert wird, bei Normaldruck und Normaltemperatur aber gasförmig ist, genügt in der Regel einfaches Verdampfen, um die organische Vorläuferverbindung auf Reaktionsstufeneingangsdruck zu bringen. Als inertes Verdünnungsgas gegebenenfalls mitverwendeter Wasserdampf steht aus den unterschiedlichsten Quellen meistens ebenfalls mit ausreichendem überatmosphärischem Druck zur Verfügung.

Dies trifft in aller Regel jedoch wenigstens nicht für die Sauerstoffquelle (z.B. Luft oder an Stickstoff entreicherte Luft), gegebenenfalls sonstige inerte Verdünnungsgase und vor allem nicht auf das Propan enthaltende Kreisgas (es weist normalerweise den Reaktionsstufeneingangsdruck $P^1$ abzüglich des Druckverlustes auf dem Weg durch die Reaktionsstufe und durch die Aufarbeitungsstufe sowie den Teiler in die beiden Teilmengen auf; seine Rückführung in die Reaktionsstufe erfolgt beim erfindungsgemäßen Verfahren normalerweise über an Einbauten freie Rohre ohne größeren zusätzlichen Druckverlust) zu.

In der Praxis ist es daher normalerweise erforderlich, wenigstens eine Teilmenge (wenigstens das Kreisgas) der Bestandteile das Reaktionsgasausgangsgemisches mittels eines Verdichters von einem niederen Ausgangsdruck auf einen höheren Enddruck (den Eingangsdruck $P^1$ in die Reaktionsstufe) zu bringen.

Dabei kann die Verdichtung dieser Bestandteile (z.B. die Sauerstoffquelle Luft und das Kreisgas) in räumlich getrennten Verdichtern oder in einem einzigen Verdichter erfolgen.

Prinzipiell können zu diesem Verdichten der genannten Gase Verdichter unterschiedlichster Art eingesetzt werden. Beispielhaft aufgeführt seien Verdrängungsverdichter (z.B. Hubkolbenverdichter, Schraubenverdichter und Drehkolbenverdichter), Strömungsverdichter (z.B. Turboverdichter, Kreiselverdichter, Axialverdichter und Radialverdichter) und Strahlverdichter. Erfindungsgemäß bevorzugt werden Radialverdichter eingesetzt, wie sie z.B. in der DE-A 10259023 beschrieben sind.

Ferner wird erfindungsgemäß bevorzugt so vorgegangen, dass man die aus verschiedenen Quellen stammenden, im wesentlichen auf Reaktionsstufeneingangsdruck befindlichen (gebrachten) Teilmengen der Reaktionsgasausgangsgemische aus getrennten Leitungen kommend zunächst meist in einem z.B. statischen Mischer (Räume mit Einbauten, die gegenüber leeren Rohren eine erhöhte Mischwirkung besitzen) vermischt und nachfolgend, gegebenenfalls auf Eingangstemperatur erwärmt, der Reaktionsstufe zuführt.

Der Eintritt der Einzelgase in die dem statischen Mischer zugeführte Leitung wird dabei in zweckmäßiger Weise häufig so gewählt, dass das Entstehen explosiver Mischungen vermieden wird (im Fall einer erfindungsgemäßen Partialoxidation kann diese Eintrittsabfolge in zweckmäßiger Weise z.B. wie folgt lauten: zunächst Kreisgas und/oder Dampf, dann Luft und schließlich die organische Vorläuferverbindung). Natürlich kann der Wasserdampfgehalt dem Reaktionsgasausgangsgemisch auch so zugegeben werden, dass man in eine durch indirekten Wärmetausch mit dem Produktgasstrom im wesentlichen auf Reaktionstemperatur erhitzte Reaktionsgasausgangsvorläufermischung fein verteilte flüssige Wassertröpfchen zudosiert, die unter Wärmeaufnahme spontan verdampfen, wobei das Reaktionsgasausgangsgemisch entsteht. Alternativ kann die vorerwärmte Reaktionsgasausgangsvorläufermischung über einen Gassättiger (Gasgemisch und Wasser werden im Gleich- oder Gegenstrom über große Oberfläche geführt) geführt werden.

Somit wirkt sich der erfindungsgemäß gewählte Ausgangsdruck $P^3$ im wesentlichen auf die Verdichterleistung zum Zweck der Verdichtung des Kreisgases und der Sauerstoffquelle aus.

Anwendungstechnisch zweckmäßig beträgt der Druck $P^3$, mit dem das Restproduktgasgemisch die Aufarbeitungsstufe beim erfindungsgemäßen Verfahren verlässt, in der Regel nicht mehr als 30 oder 25 bar, häufig nicht mehr als 20 bar. Erfindungsgemäß günstig wird der Ausgangsdruck $P^3 \geq 1{,}5$ bar und $\leq 10$ bar, vorzugsweise $\geq 2$ bar und $\leq 8$ bar, häufig $\geq 3$ bar und $\leq 6$ bar bzw. $\leq 5$ bar (z.B. 4 bar) betragen.

D.h., kennzeichnendes Merkmal des erfindungsgemäßen Verfahrens ist es, sowohl die Reaktionsstufe als auch die Aufarbeitungsstufe bei erhöhtem Druck zu betreiben.

Eine solche Verfahrensweise ist aus nachfolgenden Gründen vorteilhaft:

- überraschend wurde gefunden, dass die heterogen katalysierte partielle direkte Oxidation von Propan und/oder iso-Butan bei erhöhtem Druck unter ansonsten gleichen Reaktionsbedingungen und bezogen auf einmaligen Durchgang zu erhöhten Umsätzen führt, ohne dass damit eine wesentliche Minderung der Selektivität der Zielproduktbildung einhergeht;

- ein Betreiben auch der Aufarbeitungsstufe bei erhöhtem Druck ermöglicht ein Fördern auch erhöhter Kreisgasmengen in vergleichsweise geringen dafür erforderlichen Volumina und bei vergleichsweise geringen erlittenen Druckverlusten, da sowohl das Fördervolumen einer vorgegebenen Gasmenge als auch der mit der Förderung derselben einhergehende Druckverlust mit zunehmendem Druck in der Regel abnehmen; letzteres mindert die für die Kreisgasverdichtung auf den Eingangsdruck $P^1$ der Reaktionsstufe erforderliche Verdichterleistung; gleichzeitig minimiert eine im Vergleich zur Auslassmenge erhöhte Kreisgasmenge die Verluste an im Auslass enthaltenem, nicht umgesetztem Propan und/oder iso-Butan;

- die Rückführung des Propans ohne vorherige Abtrennung desselben aus dem Restproduktgasgemisch vermeidet die mit einer solchen Abtrennung in notwendiger Weise einhergehenden Druckverluste und gewährleistet die simultane und energetisch günstige Rückführung anderer im Restproduktgasgemisch gegebenenfalls enthaltener Bestandteile, wie z.B. Wasserdampf und $O_2$.

D.h., insgesamt lassen sich erfindungsgemäß über die vergleichsweise einfache Maßnahme einer Druckerhöhung im wesentlichen alle vorteilhaften Wesenszüge der Verfahren gemäß des Standes der Technik ebenfalls erreichen, ohne dass es dazu einer aufwendigen Abtrennung des nicht umgesetzten Alkans und gegebenenfalls Alkens aus dem Restproduktgasgemisch bedarf (was zusätzlich unerwünschte Nachteile wie z.B. eine völlige Nichtrückführung oder eine energetisch aufwendige Rückführung von Wasserdampf vermeidet), und gleichzeitig bedingt die Maßnahme der Druckerhöhung eine auf einmaligen Durchsatz durch die Reaktionsstufe bezogene Steigerung des E-duktumsatzes ohne wesentliche Minderung der Zielproduktselektivität.

Der Begriff der "Reaktionsstufe" bzw. "Aufarbeitungsstufe" steht in dieser Schrift insbesondere für eine oder mehrere hintereinandergeschaltete apparative Vorrichtungen, die gasseitig, abgesehen von Eingang und Ausgang sowie gegebenenfalls weiteren Eingängen für Hilfsgase, abgeschlossen sind, so dass sich der Druckverlust den ein Gasgemisch beim Durchgang durch eine solche apparative Vorrichtung bzw. durch eine solche Hintereinanderschaltung von apparativen Vorrichtungen erleidet auf die Überwindung der Strömungswiderstände beschränkt.

Beispielsweise kann es sich bei der apparativen Vorrichtung (oder einer Hintereinanderschaltung von solchen) um einen Rohrbündelreaktor, um einen Wirbelbettreaktor, um einen Fließbettreaktor, um eine Hintereinanderschaltung solcher Reaktoren, um eine Absorptionskolonne, um eine Rektifikationskolonne, um eine Kondensationskolonne oder um eine Hintereinanderschaltung solcher Kolonnen bzw. einzelner Quenchstufen handeln. Natürlich kann aber ein wie vorstehend beschriebener Reaktor die Möglichkeit umfassen, während der Durchführung des erfindungsgemäßen Verfahrens z.B. Katalysatoraktivator in den Reaktor zu geben, wie es z.B. die WO 02/081421 beschreibt. Der Begriff des Hilfsgases soll beim erfindungsgemäßen Verfahren z.B. die Möglichkeit mit einschließen, bei einer Hintereinanderschaltung von Reaktoren zwischen den Reaktoren Inertgas und/oder Sauerstoff (z.B. Luft) zu ergänzen, oder in der Aufarbeitungsstufe aus z.B. Polymerisationsinhibierungsgründen ein molekularen Sauerstoff enthaltendes Gas (z.B. Luft) gemeinsam mit dem Produktgasgemisch durch die Aufarbeitungsstufe zu führen. In typischer Weise betragen die Druckverluste beim erfindungsgemäßen Verfahren über die Reaktionsstufe 0,1 bis 3 bar, häufig 0,3 bis 1 oder 0,5 bar, und über die Aufarbeitungsstufe 0,5 bis 3 bar, häufig 1 bis 2 bar.

Bei der Durchführung des erfindungsgemäßen Verfahrens im Bereich besonders hoher Drucke können die Druckverluste sowohl in der Reaktionsstufe als auch in der Aufarbeitungsstufe noch deutlich tiefer liegen und z.B. Werte bis 0,05 bar und kleiner erreichen.

Der Druck $P^1$ am Eingang in die Reaktionsstufe wird beim erfindungsgemäßen Verfahren je nach Art und Weise der angewandten Aufarbeitungsstufe 0,5 oder 1 bis 4 bar, meist 1,5 bis 3,5 bar, vielfach 2 bis 3 bar oberhalb des Druckes $P^3$ am Ausgang der Aufarbeitungsstufe liegen.

Bei der Durchführung des erfindungsgemäßen Verfahrens im Bereich besonders hoher Drucke wird der Druck $P^1$ am Eingang in die Reaktionsstufe in der Regel weniger als 0,5 bar (z.B. 0,1 oder 0,01 bar) oberhalb des Druckes $P^3$ am Ausgang der Aufarbeitungsstufe liegen.

Typische Drucke $P^1$ am Eingang in die Reaktionsstufe liegen daher bei 2,5 bis 25 bar. In der Regel wird der Druck $P^1$ am Eingang in die Reaktionsstufe 3 bis 10 bar und erfindungsgemäß zweckmäßig 4 bis 8 bar betragen.

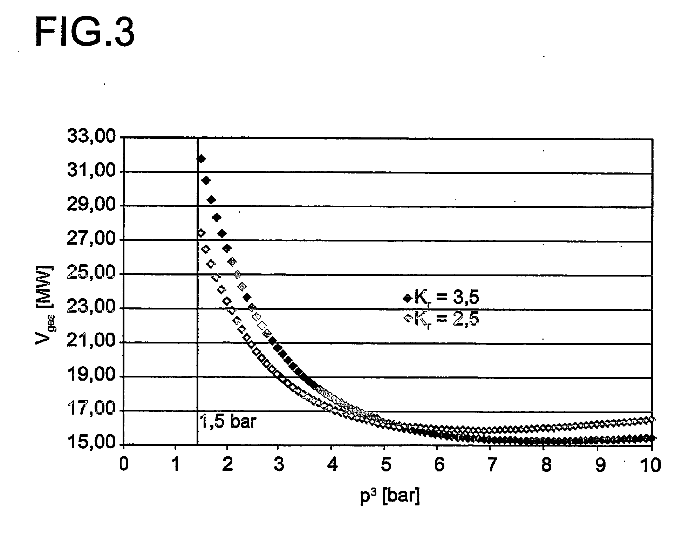

Typische Drucke $P^2$ am Eingang in die Aufarbeitungsstufe betragen 3 bis 25 bar, häu- fig 3 bis 20 bar, oder 3 bis 15 bar oder 3 bis 8 bar.

Die Regelung der Druckverhältnisse ist beim erfindungsgemäßen Verfahren in einfacher Weise z.B. mittels einer Drosselvorrichtung am Auslass für die auszuschleusende Teilmenge des Restproduktgasgemisches möglich. Mittels eines anstelle der Drosselvorrichtung nachgeschalteten Expander (inverser Verdichter, über den die Ausschleusung erfolgt) kann beim erfindungsgemäßen Verfahren im übrigen zusätzlich zu den bereits beschriebenen Vorteilen beim Auslass des einen Teils des Restproduktgasgemisches durch dessen kontrollierte Entspannung auf Normaldruck ein Teil der zur Verdichtung des im Kreis geführten anderen Teils des Restproduktgasgemisches und/oder der Sauerstoffquelle (z.B. Luft) auf den Eingangsdruck $P^1$ erforderlichen Verdichterleistung rückgewonnen werden.

Das Mengenverhältnis von derjenigen Teilmenge des Restproduktgasgemisches, die beim erfindungsgemäßen Verfahren als Kreisgas rückgeführt wird, zu derjenigen Teilmenge des Restproduktgasgemisches, die als Auslass ausgeschleust wird, hängt im Einzelnen von der Zusammensetzung des Reaktionsgasausgangsgemisches ab. In der Regel wird V jedoch ≥ 0,5 oder ≥ 1, meist ≥ 1,5, bevorzugt ≥ 2, besonders bevorzugt ≥ 3 betragen. Selbstredend kann V beim erfindungsgemäßen Verfahren auch ≥ 8 oder ≥ 10 betragen. Normalerweise wird V beim erfindungsgemäßen Verfahren ≤ 30, meist ≤ 25, häufig ≤ 20 betragen. Oft wird V ≤ 15 oder ≤ 10, bevorzugt 2 bis 8 sein.

Im übrigen kann das erfindungsgemäße Verfahren wie die aus dem Stand der Technik bekannten Verfahren der heterogen katalysierten partiellen Oxidation von Propan und/oder iso-Butan zu wenigstens einem der Zielprodukte durchgeführt werden.

D.h., als Quelle für den im Rahmen des erfindungsgemäßen Verfahrens erforderlichen molekularen Sauerstoff kommt sowohl Luft, als auch an molekularem Stickstoff entreicherte Luft (z.B. ≥ 90 Vol.%-$O_2$, ≤ 10 Vol.%-$N_2$), als auch reiner molekularer Sauerstoff oder Gemische aus molekularem Sauerstoff und anderen Inertgasen in Betracht.

Als Katalysatoren kommen für das erfindungsgemäße Verfahren prinzipiell alle diejenigen in Betracht, die im Stand der Technik für die heterogen katalysierte partielle direkte Oxidation von Propan und/oder iso-Butan zu wenigstens einem der Zielprodukte empfohlen werden.

Das sind z.B. Katalysatoren der Schriften JP-A 3-170445, EP-A 609122 und EP-A 747349.

Erfindungsgemäß wesentlich ist dabei, dass im wesentlichen alle Katalysatoren für jede der erfindungsgemäß möglichen heterogen katalysierten partiellen direkten Oxidationen eingesetzt werden können.

Bei den Aktivmassen dieser Katalysatoren handelt es sich in der Regel um Multielementoxide, meist um Multimetalloxide.

Unter den Multimetalloxiden eignen sich für das erfindungsgemäße Verfahren insbesondere die Muitimetaiioxidmassen der Schriften EP-A 608838, EP-A 529853, DE-A 10254279, DE-A 19835247, EP-A 895809, JP-A 7-232071, JP-A 11-169716, DE-A 10261186, EP-A 1192987, JP-A 10-57813, JP-A 2000-37623, JP-A 10-36311, WO 00/29105, EP-A 767164, DE-A 10029338, JP-A 8-57319, JP-A 10-28862, JP-A 11-43314, JP-A 11-574719, WO 00/29106, JP-A 10-330343, JP-A 11-285637, JP-A 310539, JP-A 11-42434, JP-A 11-343261, JP-A 3423262, WO 99/03825, JP-A 7-53448, JP-A 2000-51693, JP-A 11-263745, DE-A 10046672, DE-A 10118814, DE-A 10119933, JP-A 2000/143244, EP-A 318295, EP-A 603836, DE-A 19832033, DE-A 19836359, EP-A 962253, DE-A 10119933, DE-A 10051419, DE-A 10046672, DE-A 10033121, DE-A 101 459 58, DE-A 10122027, EP-A 1193240 und der in diesen Schriften zitierten Literatur.

Bei der zu verwendenden Aktivmasse der Katalysatorbeschickung handelt es sich in den vorgenannten Fällen im wesentlichen um Multimetalloxidmassen, die die Elemente Mo, V, wenigstens eines der beiden Elemente Te und Sb, und wenigstens eines der Elemente aus der Gruppe umfassend Nb, Ta, W, Ti, Al, Zr, Cr, Mn, Ga, Fe, Ru, Co, Cs, Ca, Sr, Ba, Rh, Ni, Pd, Pt, La, Pb, Cu, Re, Ir, Y, Pr, Nd, Tb, Bi, B, Ce, Sn, Zn, Si, Na, Li, K, Mg, Ag, Au und In in Kombination enthalten.

Bevorzugt enthält die Kombination dabei aus der letzten Elementgruppe die Elemente Nb, Ta, W und/oder Ti und besonders bevorzugt das Element Nb.

Bevorzugt enthalten die relevanten Multimetalloxidaktivmassen die vorgenannte Elementkombination in der Stöchiometrie I

$$Mo_1V_bM^1_cM^2_d \qquad (I),$$

mit

M$^1$ = Te und/oder Sb,
M$^2$ = wenigstens eines der Elemente aus der Gruppe umfassen Nb, Ta, W, Ti, Al, Zr, Cs, Ca, Sr, Ba, Cr, Mn, Ga, Fe, Ru, Co, Rh, Ni, Pd, Pt, La, Bi, Pb, Cu, Re, Ir, V, Pr, Nd, Tb, Ce, Sn, Zn, Si, Na, Li, K, Mg, Ag, Au und In,
b = 0,01 bis 1,
c = > 0 bis 1, und
d = > 0 bis 1.

Erfindungsgemäß bevorzugt ist M$^1$ = Te und M$^2$ = Nb, Ta, W und/oder Ti. Vorzugsweise ist M$^2$ = Nb.

Der stöchiometrische Koeffizient b beträgt mit Vorteil 0,1 bis 0.6. In entsprechender Weise beläuft sich der Vorzugsbereich für den stöchiometrischen Koeffizienten c auf 0,01 bis 1 bzw. auf 0,05 bis 0,4 und günstige Werte für d betragen 0,001 bis 1 bzw. 0,01 bis 0,6.

Erfindungsgemäß besonders günstig ist es, wenn die stöchiometrischen Koeffizienten b, c und d simultan in den vorgenannten Vorzugsbereichen liegen.

Das Vorgenannte gilt insbesondere dann, wenn die Aktivmasse der Katalysatorbeschickung hinsichtlich ihrer von Sauerstoff verschiedenen Elemente aus einer vorgenannten Elementkombination besteht.

Dies sind dann insbesondere die Multimetalloxidaktivmassen der allgemeinen Stöchiometrie II

$$Mo_1V_bM^1_cM^2_dO_n \qquad (II),$$

wobei die Variablen die bezüglich der Stöchiometrie I angeführte Bedeutung aufweisen und n = eine Zahl ist, die durch die Wertigkeit und Häufigkeit der von Sauerstoff verschiedenen Elemente in (II) bestimmt wird.

Bevorzugt enthalten die relevanten Multimetalloxidmassen die eingangs genannte Elementkombination in der Stöchiometrie III

$$Mo_1V_{a'}M^4_{b'}M^5_{c'}M^6_{d'} \qquad (III)$$

mit

M⁴ = wenigstens eines der Elemente aus der Gruppe umfassend Te und Sb;

M⁵ = wenigstens eines der Elemente aus der Gruppe umfassend Nb, Ti, W, Ta und Ce;

M⁶ = wenigstens eines der Elemente aus der Gruppe umfassend Pb, Ni, Co, Bi, Pd, Cs, Ca, Sr, Ba, Ag, Pt, Cu, Au, Ga, Zn, Sn, In, Re, Ir, Sm, Sc, Y, Pr, Nd und Tb;

a' = 0,01 bis 1;

b' = > 0 bis 1;

c' = > 0 bis 1; und

d' = 0 bis 0,5.

Bevorzugt beträgt a' 0,05 bis 0,6, besonders bevorzugt 0,1 bis 0,6 oder 0,5. Bevorzugt beträgt b' 0,01 bis 1, besonders bevorzugt 0,01 bzw. 0,1 bis 0,5 oder 0,4. Bevorzugt beträgt c' 0,01 bis 1, besonders bevorzugt 0,01 bzw. 0,1 bis 0,5 oder 0,4. Bevorzugt beträgt d' 0,00005 bzw. 0,0005 bis 0,5, besonders bevorzugt 0,001 bis 0,5, häufig 0,002 bis 0,3 und oft 0,005 bzw. 0,01 bis 0,1.

M⁴ ist bevorzugt Te.

M⁵ ist vorzugsweise wenigstens zu 50 mol.-% seiner Gesamtmenge Nb, bevorzugt zu wenigstens 75 mol.-% und ganz besonders bevorzugt zu wenigstens 100 mol.-%.

M⁶ ist bevorzugt wenigstens ein Element aus der Gruppe umfassend Ni, Co, Bi, Pd, Ag, Au, Pb und Ga, besonders bevorzugt wenigstens ein Element aus der Gruppe umfassend Ni, Co, Pd und Bi.

Ganz besonders bevorzugt ist M⁵ wenigstens zu 50 mol.-%, oder zu wenigstens 75 mol.-%, oder zu 100 mol.-% seiner Gesamtmenge Nb und M⁶ wenigstens ein Element aus der Gruppe umfassend Ni, Co, Pd und Bi.

Erfindungsgemäß herausragend ist M⁴ = Te, M⁵ = Nb und M⁶ wenigstens ein Element aus der Gruppe umfassend Ni, Co und Pd.

Das Vorgenannte gilt insbesondere dann, wenn die Aktivmasse der Katalysatorbeschickung hinsichtlich ihrer von Sauerstoff verschiedenen Elemente aus einer Elementkombination der Stöchiometrie (III) besteht. Das sind dann insbesondere die Mulitmetalloxidaktivmassen der allgemeinen Stöchiometrie (IV)

$$Mo_1V_{a'}M^4_{b'}M^5_{c'}M^6_{d'}O_{n'} \qquad (IV),$$

wobei die Variablen die bezüglich der Stöchiometrie III angeführte Bedeutung aufweisen und n' eine Zahl ist, die durch die Wertigkeit und Häufigkeit der von Sauerstoff verschiedenen Elemente in (IV) bestimmt wird.

Ferner werden für das erfindungsgemäße Verfahren bevorzugt solche Multimetalloxidaktivmassen eingesetzt, die einerseits entweder eine der vorgenannten Elementkombinationen (I), (III) enthalten oder, bezüglich der von Sauerstoff verschiedenen Elemente, aus ihr bestehen und jeweils gleichzeitig ein Röntgendiffraktogramm aufweisen, das Beugungsreflexe h und i zeigt, deren Scheitelpunkte bei den Beugungswinkeln (2Θ) 22,2 ± 0,5° (h) und 27,3 ± 0,5° (i) liegen (alle in dieser Schrift auf ein Röntgendiffraktogramm bezogenen Angaben beziehen sich auf ein unter Anwendung von Cu-Kα-Strahlung als Röntgenstrahlung erzeugtes Röntgendiffraktogramm (Siemens-Diffraktometer Theta-Theta D-5000, Röhrenspannung: 40 kV, Röhrenstrom : 40mA, Aperturblende V20 (variabel), Streustrahlblende V20 (variabel), Sekundärmonochromatorblende (0,1 mm), Detektorblende (0,6 mm), Meßintervall (2Θ): 0,02°, Meßzeit je Schritt: 2,4s, Detektor: Scintillationszählrohr).

Die Halbwertsbreite dieser Beugungsreflexe kann dabei sehr klein oder auch sehr ausgeprägt sein.

Günstig sind für das erfindungsgemäße Verfahren diejenigen der vorgenannten Multimetalloxidaktivmassen, deren Röntgendiffraktogramm zusätzlich zu den Beugungsreflexen h und i einen Beugungsreflexes k aufweist, dessen Scheitelpunkt bei 28,2 ± 0,5° (k) liegt.

Unter den letzteren sind wiederum jene erfindungsgemäß bevorzugt, bei denen der Beugungsreflex h innerhalb des Röntgendiffraktogramms der intensitätsstärkste ist, sowie eine Halbwertsbreite von höchstens 0,5° aufweist, und ganz besonders bevorzugt sind jene, bei denen die Halbwertsbreite des Beugungsreflexes i und des Beugungsreflexes k gleichzeitig jeweils ≤ 1° beträgt und die Intensität $P_k$ des Beugungsreflexes k und die Intensität $P_i$ des Beugungsreflexes i die Beziehung 0,2 ≤ R ≤ 0,85, besser 0,3 ≤ R ≤ 0,85, bevorzugt 0,4 ≤ R ≤ 0,85, besonders bevorzugt 0,65 ≤ R ≤ 0,85, noch mehr bevorzugt 0,67 ≤ R ≤ 0,75 und ganz besonders bevorzugt R = 0,70 bis 0,75 bzw. R = 0,72 erfüllen, in der R das durch die Formel

$$R = P_i/(P_i+P_k)$$

definierte Intensitätsverhältnis ist. Bevorzugt weisen die vorgenannten Röntgendiffraktogramme keinen Beugungsreflex auf, dessen Maximum bei 2 Θ = 50 ± 0,3° liegt.

Die Definition der Intensität eines Beugungsreflexes im Röntgendiffraktogramm bezieht sich in dieser Schrift auf die in der DE-A 19835247, der DE-A 10122027, sowie die in der DE-A 10051419 und DE-A 10046672 niedergelegte Definition. Das gleiche gilt für die Definition der Halbwertsbreite.

Neben den Beugungsreflexen h, i und k enthalten die vorgenannten Röntgendiffraktogramme von solchen erfindungsgemäß vorteilhaft einzusetzenden Multimetalloxidaktivmassen noch weitere Beugungsreflexe, deren Scheitelpunkte bei den nachfolgenden Beugungswinkeln (2 Θ) liegen:

$$9,0 \pm 0,4^o \text{ (l)}$$

$$6,7 \pm 0,4^o \text{ (o)}$$

und

$$7,9 \pm 0,4^o \text{ (p)}.$$

Günstig ist es ferner, wenn das Röntgendiffraktogramm zusätzlich einen Beugungsreflex enthält, dessen Scheitelpunkt beim Beugungswinkel (2 Θ) = 45,2 ± 0,4° (q) liegt.

Häufig enthält das Röntgendiffraktogramm auch noch die Reflexe 29,2 ± 0,4° (m) und 35,4 ± 0,4° (n).

Es ist ferner günstig, wenn die in den Formeln (I), (II), (III) und (IV) definierten Elementkombinationen als reine i-Phase vorliegen. Enthält die katalytisch aktive Oxidmasse auch noch k-Phase, enthält ihr Röntgendiffraktogramm neben den oben genannten noch weitere Beugungsreflexe, deren Scheitelpunkte bei den nachfolgenden Beugungswinkeln (2 Θ) liegen: 36,2 ± 0,4° und 50 ± 0,4° (die Begriffe i- und k-Phase werden in dieser Schrift wie in der DE-A 10122027 und DE-A 10119933 festgelegt verwendet).

Ordnet man dem Beugungsreflex h die Intensität 100 zu, ist es erfindungsgemäß günstig, wenn die Beugungsreflexe i, l, m, n, o, p, q in der gleichen Intensitätsskala die nachfolgenden Intensitäten aufweisen:

i: 5 bis 95, häufig 5 bis 80, teilweise 10 bis 60;
l: 1 bis 30;
m: 1 bis 40;
n: 1 bis 40;
o: 1 bis 30;
p: 1 bis 30 und
q: 5 bis 60.

Enthält das Röntgendiffraktogramm von den vorgenannten zusätzlichen Beugungsreflexen, ist die Halbwertsbreite derselben in der Regel ≤ 1°.

Die spezifische Oberfläche von erfindungsgemäß zu verwendenden Multimetalloxidaktivmassen der allgemeinen Formel (II) oder (IV) oder von Multimetalloxidaktivmassen, die Elementkombinationen der allgemeinen Formel (I) oder (III) enthalten beträgt vielfach 1 bis 40 $m^2/g$ bzw. 10 bis 30 $m^2/g$ (BET-Oberfläche, Stickstoff), vor allem dann, wenn ihr Röntgendiffraktogramm wie beschrieben gestaltet ist.

Die Herstellung der beschriebenen Multimetalloxidaktivmassen findet sich im im Zusammenhang mit diesen zitierten Stand der Technik. Dazu zählen insbesondere die DE-A 10303526, die DE-A 10261186, die DE-A 10254279, die DE-A 10254278, die DE-A 10122027, die DE-A 10119933, DE-A 10033121, die EP-A 1192987, die DE-A 10029338, die JP-A 2000-143244, die EP-A 962253, die EP-A 895809, die DE-A 19835247, die WO 00/29105, die WO 00/29106, die EP-A 529853 und die EP-A 608838 (in allen Ausführungsbeispielen der beiden letztgenannten Schriften ist als Trocknungsmethode die Sprühtrocknung anzuwenden; z.B. mit einer Eingangstemperatur von 300 bis 350°C und einer Ausgangstemperatur von 100 bis 150°C; Gegenstrom oder Gleichstrom).

Die beschriebenen Multimetalloxidaktivmassen können als solche (d.h. in Pulverform) oder zu geeigneten Geometrien geformt (z.B. wie die Schalenkatalysatoren der DE-A 10051419 oder die geometrischen Varianten der DE-A 10122027) für das erfindungsgemäße Verfahren eingesetzt werden. Sie eignen sich hervorragend zur Herstellung von Acrylsäure aus Propan, aber auch zur Herstellung von Methacrylsäure aus iso-Butan.

Zur Durchführung des erfindungsgemäßen Verfahrens können alle genannten Katalysatoren sowohl unverdünnt, als auch mit inerten Partikeln und/oder Formkörpern (sie weisen keine Aktivmasse auf) verdünnt eingesetzt werden. Geeignetes Verdünnungsmaterial ist z.B. Steatit.

Die Geometrie der Verdünnungsformkörper ist dabei mit derjenigen der Katalysatorformkörper vorzugsweise identisch.

Wie in den Schriften über für das erfindungsgemäße Verfahren geeignete Multimetalloxidaktivmassekatalysatoren beschrieben, kann das erfindungsgemäße Verfahren sowohl an Festbettkatalysatorbeschickungen als auch an Wirbelbett- oder Fließbettkatalysatorbeschickungen durchgeführt werden. Die dabei erfindungsgemäß anwendbaren Reaktionsstufeeingangsdrucke wurden bereits beschrieben.

Die Reaktionstemperaturen, insbesondere bei Anwendung der in dieser Schrift empfohlenen Katalysatoren, kann 200 bis 700°C, bevorzugt 200 bis 550°C oder 230 bis 480°C oder 300 bis 440°C betragen.

Die Belastung der Katalysatorbeschickung mit Propan und/oder iso-Butan kann 50 bis 3000 NI/I (Katalysatorbeschickung)/h, oder 80 bis 1500 NI/I/h, oder 100 bis 1000 NI/I/h, oder 120 bis 600 NI/I/h, oder 140 bis 300 NI/I/h betragen.

Die Belastung mit Reaktionsgasausgangsgemisch der Katalysatorbeschickung kann dabei 100 bis 10000 NI/I/h, oder 300 bis 6000 NI/I/h, oder 300 bis 2000 NI/I/h betragen. Die mittlere Verweilzeit in der Katalysatorbeschickung kann 0,01 bis 10 s, oder 0,1 bis 10 s, oder 2 bis 6 s betragen.

Im Fall einer Herstellung von Acrylsäure aus Propan bzw. von Methacrylsäure aus iso-Butan kann das Reaktionsgasausgangsgemisch z.B. enthalten:

0,5 bis 15, häufig 1 bis 7 Vol.-% Propan, bzw. iso-Butan
10 bis 90, häufig 20 bis 50 Vol.-% Luft,
0 bis 50 Vol.-% Wasserdampf und

als Restmenge Kreisgas.

Das Reaktionsgasausgangsgemisch kann im Fall einer Herstellung von Acrylsäure aus Propan bzw. von Methacrylsäure aus iso-Butan für das erfindungsgemäße Verfahren aber auch enthalten:

0,6 bis 1,2 Vol.-% Propan, bzw. iso-Butan,
65 bis 95 Vol.-% Luft,
2 bis 30 Vol.-% Stickstoff,
0,05 bis 0,8 Vol.-% $CO_x$ und
2 bis 3 Vol.-% Wasserdampf.

Bevorzugt sind Reaktionsgasausgangsgemische, die 10 bis 50 Vol.-% Wasserdampf (frisch) enthalten.

Eine andere mögliche Zusammensetzung des Reaktionsgasausgangsgemisches kann z.B. enthalten
70 bis 90 Vol.-% Propan oder iso-Butan,
5 bis 25 Vol.-% molekularer Sauerstoff,
0 bis 25 Vol.-% Wasserdampf und
als Restmenge Kreisgas.

Bevorzugt sind auch hier Reaktionsgasausgangsgemische, die insgesamt 10 bis 50 Vol.-% Wasserdampf enthalten. D.h., in typischer Weise bewegt sich die Zusammensetzung des Reaktionsgasausgangsgemisches für das erfindungsgemäße Verfahren im Fall einer Propan- oder iso-Butanpartialoxidation innerhalb des folgenden Rahmens (molare Verhältnisse):

| iso-Butan oder Propan | : | Sauerstoff | : | $H_2O$ | : | sonstige Verdünnungsgase = |
|---|---|---|---|---|---|---|
| 1 | : | (0,1-10) | : | (0-50) | : | (0-50), |

(fortgesetzt)

| vorzugsweise | 1 | : | (0,1-10) | : | (0,1-50) | : | (1-50), |
| besonders bevorzugt | 1 | : | (0,5-5) | : | (1-30) | : | (1-30). |

Die vorgenannten Bereich gelten insbesondere dann, wenn als sonstige Verdünnungsgase überwiegend molekularer Stickstoff eingesetzt wird. Andere mögliche Verdünnungsgase sind z.B. He, Ar, CO und/oder $CO_2$ etc.

Ganz allgemein wird das Reaktionsgasausgangsgemisch in seiner Zusammensetzung so gewählt, dass es vorzugsweise außerhalb des Explosionsgasbereiches liegt.

Wird das erfindungsgemäße Verfahren als partielle Oxidation durchgeführt, kann dies z.B. in Einzonen-Rohrbündelreaktoren erfolgen, wie sie in der EP-A 700714 und in der EP-A 700893 beschrieben sind. Es kann aber auch in Mehrzonen-Rohrbündelreaktoren ausgeführt werden, wie sie in der DE-A 19927624, der DE-A 19948242, der DE-A 19948241, der DE-A 19910508 und in der DE-A 19910506 beschrieben sind. Eine Durchführung des erfindungsgemäßen Verfahrens im Wirbelbett kann z.B. wie in der WO 02/0811421 beschrieben durchgeführt werden.

Bezogen auf das im Reaktionsgasausgangsgemisch enthaltene Propan und/oder iso-Butan wird der Umsatz an Propan und/oder iso-Butan beim erfindungsgemäßen Verfahren, bezogen auf einmaligen Durchgang des Reaktionsgasgemisches durch die Reaktionsstufe, in der Regel 10 oder 20 bis 70 mol-%, häufig 30 bis 60 mol-% und vielfach 40 bis 60 mol-% bzw. 45 bis 55 mol-% betragen. Die Selektivität der Zielproduktbildung wird üblicherweise 40 bis 98 oder 95 oder bis 90 mol-%, vielfach 50 bis 80 mol-%, oft 60 bis 80 mol-% betragen.

Die Grundabtrennung des wenigstens einen Zielproduktes vom in der Reaktionsstufe des erfindungsgemäßen Verfahrens resultierenden Produktgasgemisch kann in der erfindungsgemäßen Aufarbeitungsstufe prinzipiell so durchgeführt werden, wie es aus den Verfahren gemäß Stand der Technik bekannt ist. Insbesondere können erfindungsgemäß auch die Aufarbeitungsverfahren angewendet werden, die im Stand der Technik für die Grundabtrennung derselben Zielprodukte aus Produktgasgemischen bekannt sind, wie sie bei der Herstellung der Zielprodukte durch heterogen katalysierte partielle Oxidation von Propen und/oder iso-Buten bekannt sind.

In der Regel wird man das beim erfindungsgemäß Verfahren in der Reaktionsstufe resultierende Produktgasgemisch bei Eintritt in die erfindungsgemäße Aufarbeitungsstufe zunächst einer indirekten und/oder direkten Abkühlung unterwerfen.

Zum Zweck der Grundabtrennung von im Produktgasgemisch enthaltenem Zielprodukt in eine Flüssigphase kann das so abgekühlte (im Fall der Acrylsäure z.B. typisch auf 150 - 250°C) oder auch nicht abgekühlte Produktgasgemisch z.B. in einer Absorptionskolonne im Gegenstrom zu absteigendem flüssigem Absorptionsmittel geführt werden, das das wenigstens eine Zielprodukt im wesentlichen selektiv aus dem Produktgasgemisch absorbiert, wie es z.B. die JP-A 2001/0026269, die EP-A 990636, die JP-A 2000/327651, die EP-A 925272, die EP-A 695736, die EP-A 778255, die DE-A 2136396, die DE-A 2449780, die DE-A 4308087, die EP-A 982287, die EP-A 982289, die EP-A 982288 und die DE-A 19631645 für die Zielprodukte und verschiedene Absorptionsmittel beschreiben.

Für im wesentlichen beide Zielprodukte kommen als Absorptionsmittel sowohl Wasser (oder wässrige Mischungen, z.B. wässrige Natronlauge oder wässrige Acrylsäure bzw. Methacrylsäure), zur Veresterung von Acryl- und Methacrylsäure verwendete Alkohole wie z.B. 2-Ethylhexanol, als auch höher siedende organische Lösungsmittel in Betracht. Erfindungsgemäß bevorzugt liegt der Siedepunkt des organischen Lösemittels wenigstens 20°C, insbesondere wenigstens 50°C und besonders bevorzugt wenigstens 70°C über dem Siedepunkt des aus dem Produktgasgemisch abzutrennenden Zielprodukts (Acrylsäure und/oder Methacrylsäure). Erfindungsgemäß bevorzugte organische Absorptionsmittel haben Siedepunkte (bei Normaldruck) von 180 bis 400°C, insbesondere von 220 bis 360°C. Erfindungsgemäß besonders geeignete Absorptionsmittel sind im Fall der Zielprodukte Acrylsäure und Methacrylsäure hochsiedende, extrem hydrophobe Lösungsmittel, die keine nach außen wirkende polare Gruppe enthalten, wie aliphatische oder aromatische Kohlenwasserstoffe, z.B. Mittelölfraktionen aus der Paraffindestillation, oder Äther mit sperrigen Gruppen am O-Atom, oder Gemische davon, wobei diesen vorteilhafterweise ein polares Lösungsmittel, wie das in der DE-A 4308087 offenbarte 1,2-Dimethylphthalat zugesetzt wird. Weiterhin eignen sich Ester der Benzoesäure und Phthalsäure mit geradkettigen, 1 bis 8 Kohlenstoffatome enthaltenden Alkanolen, wie Bezoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkane, z.B. 4-Methyl-4'-benzyldiphenylmethan und dessen Isomere 2-Methyl-2'-benzyl-diphenylmethan, 2-Methyl-4'-benzyl-diphenylmethan und 4-Methyl-2'-benzyl-diphenylmethan und Gemische solcher Isomerer.

Ein für Acrylsäure besonders bevorzugtes Absorptionsmittel (Methacrylsäure wird vorzugsweise in Wasser absorbiert) ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, bezogen auf 100 Gew.-% Diphenyl und Diphenylether, beispielsweise das im Handel erhältliche Diphyl®. Vorzugsweise enthält dieses Lösungsmittelgemisch weiterhin ein polares Lösungsmittel wie Dimethylphthalat in einer Menge von 0,1 bis 25 Gew.-%, bezogen auf das gesamte Lösungsmittelgemisch. Vorteilhafterweise wird das Produktgasgemisch bei Verwendung eines hochsiedenden organischen Lösungsmittels als Absorptionsmittel vor der Absorption durch Teilverdampfen des Absorptionsmittels in einem Direktkondensator oder Quenchapparat gekühlt. Hierfür eignen sich insbesondere Venturiwäscher, Blasensäulen oder Sprühkondensatoren.

Die Begriffe Hoch-, Schwer-, Mittel- und Leichtsieder meinen in dieser Schrift Verbindungen, die einen höheren Siedepunkt als die Zielverbindung (Hochsieder), etwa den gleichen Siedepunkt wie die Zielverbindung (Mittelsieder) bzw. einen niedrigeren Siedepunkt als die Zielverbindung (Leichtsieder) besitzen. Insbesondere dann, wenn die Zielverbindung Acrylsäure ist.

Ganz generell erfolgt die Gegenstromabsorption bevorzugt in einer Kolonne mit geordneten Packungen oder regellosen Füllkörperschüttungen oder in einer Bodenkolonne, die vorzugsweise mit Dual-flow-böden und/oder Ventilböden bestückt ist und die von oben mit Lösungsmittel beaufschlagt wird. Das Produktgasgemisch (und gegenbenenfalls verdampftes Absorptionsmittel aus dem Quenchapparat) wird von unten in die Kolonne eingeleitet und anschließend auf Absorptionstemperatur gekühlt. Die Abkühlung erfolgt vorteilhafterweise durch Kühlkreise. D.h., erwärmtes, in der Kolonne absteigendes Absorptionsmittel wird aus der Kolonne abgezogen, in Wärmetauschern abgekühlt und wieder an einer Stelle oberhalb der Abzugsstelle in die Absorptionskolonne rückgeführt. Nach der Absorption befinden sich im wesentlichen alle Schwersieder, der größte Teil der Zielverbindung (z.B. Acrylsäure) und ein Teil der Leichtsieder im Absorptionsmittel. Aus dem die Zielverbindung (z.B. Acrylsäure) grundabgetrennt enthaltenden Absorbat kann dann das Zielprodukt wie im Stand der Technik (z.B. dem bei der Absorption zitierten) beschreiben in beliebiger Reinheit weiter abgetrennt (z.B. wie in der DE-A 19606877 oder in der DE-A 19838845) und vom Zielprodukt befreites Absorptionsmittel in der Absorption wieder verwendet werden (z.B. aus dem organischen Absorbat die Acrylsäure über Kopf vom organischen Absorptionsmittel abtrennen und rektifikativ und/oder kristallisativ (z.B. Suspensionskristallisation mit Kristallisatabtrennung in einer Schmelzewaschkolonne) weiterreinigen oder aus dem wässrigen Absorbat das Wasser mittels eines organischen Schleppmittels rektifikativ über Kopf und die Acrylsäure aus dem Acrylsäure enthaltenden Sumpf rektifikativ und/oder kristallisativ in beliebiger Reinheit weiterabtrennen; im letzteren Fall wird das Kopfprodukt in der Regel in zwei Phasen geschieden (durch Kühlen); die organische Phase wird in die Rektifikationskolonne und die wässrige Phase in die Absorptionskolonne rückgeführt (jeweils am Kopf der Kolonne)).

Das verbleibende, nicht absorbierte Restproduktgasgemisch kann weiter abgekühlt werden, um den einfach kondensierbaren Teil der leichtsiedenden Nebenkomponenten (z.B. Wasser, Formaldehyd und Essigsäure) abzutrennen (in der Regel Sauerwasser genannt). Das verbleibende Restproduktgasgemisch kann erfindungsgemäß in zwei Portionen aufgeteilt und die eine der beiden Portionen als Kreisgas rückgeführt (in die Reaktionsstufe) und die andere Portion ausgelassen werden. Bevorzugt erfolgt erfindungsgemäß keine Sauerwasserabtrennung. Insbesondere wenn in der Reaktionsstufe des erfindungsgemäßen Verfahrens Wasserdampf als Verdünnungsgas mitverwendet (bei Verwendung von Multimetalloxidmassen (I), (II), (III) oder (IV) ist dies in der Regel für die Selektivität der Zielproduktbildung vorteilhaft) wird, erfolgt die erfindungsgemäße Grundabtrennung von im Produktgasgemisch der Reaktionsstufe enthaltenem Zielprodukt in die Flüssigphase (unabhängig davon, welches, insbesondere von den in dieser Schrift beschriebenen, Trennverfahren angewendet wird) bevorzugt so, dass im verbleibenden, erfindungsgemäß wenigstens als Teilmenge (als Kreisgas) in die Reaktionsstufe rückzuführenden, Restproduktgasgemisch das molare Verhältnis W des darin enthaltenen Wasserdampf zum darin enthaltenen Propan höchstens 50 %, besser höchstens 40 oder 30 %, noch besser höchstens 20 oder 10 %, bevorzugt höchstens 5 %, kleiner ist, als das entsprechende molare Verhältnis W' im Produktgasgemisch der Reaktionsstufe. Im Extremfall können beim erfindungsgemäßen Verfahren die vorgenannten Verhältnisse W und W' auch identisch sein.

Der Versuch, im Restproduktgasgemisch möglichst viel Wasserdampf zu belassen, verfolgt hier den Zweck, auf eine Frischzufuhr (bzw. Kondensation und Neuverdampfung) von Wasserdampf in das Reaktionsgasausgangsgemisch möglichst weitgehend verzichten zu können.

Allerdings muß beim erfindungsgemäßen Verfahren die Teilmenge des Restproduktgasgemisches, die als Auslaß ausgeschleust wird, wenigstens soviel Wasser enthalten, wie in der Reaktionsstufe als Nebenprodukt gebildet wird, um ein Aufpegeln des Wassers im Reaktionsgasgemisch zu verhindern (je selektiver die Reaktion in der Reaktionsstufe geführt wird, desto geringer ist die auszulassende Wassermenge). Dies gilt in entsprechender Weise auch für andere in der Reaktionsstufe gebildeten Nebenkomponenten. Wird beim erfindungsgemäßen Verfahren Luft als Sauerstoffquelle verwendet, muß die Auslassmenge an Restproduktgasgemisch gleichzeitig so bemessen werden, dass die darin enthaltene Stickstoffmenge wenigstens derjenigen entspricht, die in der Luftzufuhr enthalten ist.

Wird als Absorptionsmittel eines der hochsiedenden organischen Lösungsmittel verwendet, wird man die Absorption im vorstehenden Fall bevorzugt so durchführen (insbesondere im Fall einer Absorption von Acrylsäure), dass der Ablauf aus der Absorptionskolonne einphasig ist. Im übrigen lässt sich der Wasserdampfgehalt im bei der Absorption verbleibenden Restproduktgasgemisch, unabhängig vom gewählten Absorptionsmittel, durch geeignete Wahl der Absorptionstemperatur einstellen.

Alternativ zu einer Grundabtrennung von Zielprodukt in eine Flüssigphase durch Absorption in ein Lösungsmittel, kann diese Grundabtrennung (insbesondere im Fall von Acrylsäure) auch durch Kondensation, insbesondere fraktionierende Kondensation, erfolgen, wie es z.B. die DE-A 19924532, die DE-A 10200583, die DE-A 10053086, die DE-A 19627847, die DE-A 19740253, die DE-A 19740252, die DE-A 19740253, die DE-A 19814387 und die DE-A 10247240 beschreiben.

Dabei wird das Produktgasgemisch der Reaktionsstufe, gegebenenfalls nach direkter und/oder indirekter Vorkühlung, in einer mit trennwirksamen Einbauten versehenen Trennkolonne in sich selbst aufsteigend einer fraktionierenden Kondensation unterworfen und das Zielprodukt über einen Seitenabzug der Trennkolonne entnommen und wie im Stand der Technik beschrieben weiteren kristallisativen und/oder rektifikativen Trennschritten unterworfen.

Als trennwirksame Einbauten enthaltende Kondensationskolonne sind dabei Bodenkolonnen bevorzugt, die von unten nach oben zunächst Dual-Flow-Böden und im Anschluß daran hydraulisch abgedichtete Querstromböden als trennwirksame Einbauten enthalten, wie sie im vorgenannten Stand der Technik beschrieben werden.

Erfindungsgemäß bevorzugt wird auch die vorgenannte fraktionierende Kondensation so durchgeführt, dass im wesentlichen keine Abtrennung von im Produktgasgemisch enthaltenem Wasser erfolgt. D.h., vorzugsweise erfolgt auch hier keine Sauerwasserabtrennung.

Allerdings kann die als Auslass ausgeschleuste Teilmenge des Restproduktgasgemisches vorab ihres Auslass noch mit Wasser gewaschen werden, um Methacrylsäure- bzw. Acrylsäureverluste zu vermeiden. Aus dem dabei resultierenden Methacrylsäure bzw. Acrylsäure haltigen Sauerwasser kann die Acrylsäure bzw. Methacrylsäure und gegebenenfalls andere organische Wertprodukte z.B. mit dem zur Absorption verwendeten organischen Absorptionsmittel extrahiert und mit dem Absorbat vereinigt werden.

Sowohl die Absorptionskolonne als auch die Kolonne zur fraktionierenden Kondensation kann, zum Zweck der Vermeidung erhöhter Druckverluste, prinzipiell auch durch eine Hintereinanderschaltung von Quenchstufen ersetzt werden, die mit Absorptionsmittel oder Kondensat betrieben werden.

Ganz generell erfolgt die Polymerisationsinhibierung im Rahmen der Grundabtrennung von Zielprodukt aus dem Produktgasgemisch in eine Flüssigphase wie im Stand der Technik beschrieben durch Zusatz der entsprechenden Polymerisationsinhibitoren.

Erfindungsgemäß wesentlich ist, dass das bei der Grundabtrennung von Zielprodukt verbleibende Restproduktgasgemisch neben Propan und/oder iso-Butan sowie gegebenenfalls Propen und/oder iso-Buten normalerweise wenigstens 5 Vol.-%, meist wenigstens 10 Vol.-% an bei Normaldruck leichter als das Zielprodukt siedenden Bestandteilen enthält. Dies sind insbesondere die bei Normaldruck leichter als Wasser siedenden Bestandteile (z.B. $N_2$, CO, $CO_2$) des Produktgasgemisches sowie vorzugsweise Wasser selbst.

Ein Vorteil des erfindungsgemäßen Verfahrens besteht darin, dass das Kreisgas in der Regel mittels eines Gebläses (ca. vom Ausgangsdruck $P^3$) auf den Eingangsdruck $P^1$ rückverdichtet werden kann. Unter einem Gebläse wird dabei ein Verdichter (normalerweise ein Axialverdichter) mit niedrigem Druckverhältnis (Enddruck zu Ausgangsdruck wie 1,1 : 1 bis 3 : 1) verstanden. Im Unterschied dazu wird als Sauerstoffquelle verwendete Luft normalerweise mittels eines Radialverdichters (ca. vom Umgebungsdruck) auf den Eingangsdruck $P^1$ verdichtet. Prinzipiell können die in dieser Schrift angesprochenen Verdichtungen isotherm oder polytrop durchgeführt werden. Letzteres ist erfindungsgemäß bevorzugt.

Ein Teil der Vorteilhaftigkeit der erfindungsgemäßen Verfahrensweise bleibt auch dann noch erhalten, wenn man aus dem auszuschleusenden Teil des Restproduktgasgemisches, gegebenenfalls nach vorheriger kondensativer Abtrennung des darin enthaltenen Wasseranteils (kann in die Reaktionsstufe rückgeführt werden), das darin enthaltene Propan und/oder iso-Butan sowie gegebenenfalls Propen und/oder iso-Buten abtrennt und auf den Eingangsdruck $P^1$ rückverdichtet in die Reaktionsstufe rückführt.

Diese Abtrennung (die im Stand der Technik für die Gesamtmenge des Restproduktgasgemisches empfohlen wird und prinzipiell auch (insbesondere wie nachfolgend beschrieben) angewendet werden kann) wird man zum Beispiel so vornehmen (vgl. WO 0196271), dass man das im auszuschleusenden Teil des Restproduktgasgemisches enthaltene Propan und/oder iso-Butan sowie gegebenenfalls Propen und/oder iso-Buten absorptiv und/oder adsorptiv abtrennt und durch Desorbtion und/oder Desorption nachfolgend wieder freisetzt. Beispielsweise kann diese Freisetzung aus dem Absorbat durch Strippen mit Luft durchgeführt werden, wie es z.B. die WO 0196271 beschreibt. Die Luft kann nachfolgend mitverdichtet und in die Reaktionsstufe mitrückgeführt werden.

Alternativ kann der auszuschleusende Teil des Restproduktgasgemisches auch einem weiteren Oxidationsreaktor, gegebenenfalls nach Ergänzung von Sauerstoff, zugeführt werden, um den Kohlenwasserstoffumsatz zu erhöhen.

In der Regel ist, wie bereits gesagt, beim erfindungsgemäßen Verfahren zum Zweck der Rückverdichtung des Kreisgases ein Gebläse ausreichend.

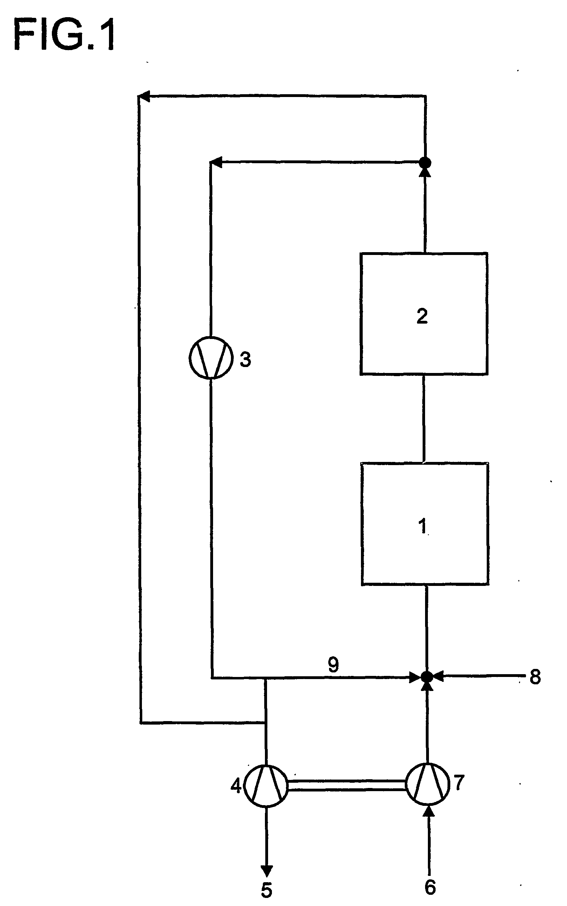

Die dieser Schrift beiliegende Figur 1 zeigt eine beispielhafte Ausführungsvariante des erfindungsgemäßen Verfahrens.

Dabei haben die verschiedenen Ziffern die nachfolgende Bedeutung:

1 = Reaktionsstufe
2 = Aufarbeitungsstufe

3 = Kreisgasverdichter (Gebläse)
4 = Auslassexpander
5 = Auslaß
6 = Luft als Sauerstoffquelle
7 = Luftverdichter
8 = frisches Propan und/oder iso-Butan sowie gegebenenfalls Wasserdampf
9 = rückverdichtetes Kreisgas

Beispiele

1. Herstellung eines Katalysators mit der Multimetalloxidaktivmasse $Mo_1V_{0,29}Te_{0,13}Nb_{0,13}O_x$ (reine i-Phase)

87,61 g Ammoniummetavanadat (78,55 Gew.-% $V_2O_5$, Fa. G.f.E., Nürnberg) wurden bei 80°C in 3040 ml Wasser gelöst (Dreihalskolben mit Rührer, Thermometer und Rückflusskühler). Es entstand eine klare gelbliche Lösung. Diese Lösung wurde auf 60°C abgekühlt und dann unter Aufrechterhaltung der 60°C nacheinander 117,03 g Tellursäure (99 Gew.-% $H_6TeO_6$, Fa. Aldrich) und 400,00 g Ammoniumheptamolybdat (82,52 Gew.-% $MoO_3$, Fa. Starck, Goslar) eingerührt. Die resultierende tiefrote Lösung wurde auf 30°C abgekühlt (Lösung A).
In einem Becherglas wurden separat 112,67 g Ammoniumnioboxalat (20,8 Gew.-% Nb, Fa. Starck, Goslar) bei 60°C in 500 ml Wasser gelöst (Lösung B).
Lösung B wurde auf 30°C abgekühlt und bei dieser Temperatur mit Lösung A vereinigt, wobei die Lösung B zur Lösung A gegeben wurde. Die Zugabe erfolgte kontinuierlich über einen Zeitraum von ca. 5 Minuten. Es entstand eine orange wässrige Suspension mit schwebendem Niederschlag. Diese Suspension wurde anschließend sprühgetrocknet ($T_{Vorlagebehälter}$ = 30°C, $T^{ein}$ = 320°C, $T^{aus}$ = 110°C, t = 1,5 h, Sprühturm der Fa. Niro vom Typ Atomizer). Das resultierende Sprühgut war ebenfalls orange und enthielt die Einwaagestöchiometrie $Mo_1V_{0,33}Te_{0,22}Nb_{0,11}$.
2 x 100 g des Sprühpulvers wurden in einem Drehkugelofen gemäß Figur 1 der DE-A 10119933 (1 l Innenvo-lumen) thermisch behandelt, indem zunächst über 27,5 min mit linearer Heizrampe unter einem Luftstrom von 50 Nl/h von 25°C auf 275°C aufgeheizt und anschließend diese Temperatur für 1 h gehalten wurde. Danach wurde mit linearer Heizrampe innerhalb von 32,5 min von 275°C auf 600°C aufgeheizt, wobei der Luftstrom durch einen 50 Nl/h Stick-stoffstrom ersetzt wurde. Die 600°C wurden unter Beibehalt des Stickstoffstroms für 2 h gehalten und nachfolgend der gesamte Ofen auf Raumtemperatur abkühlen gelassen. 100 g der erhaltenen oxidischen Masse wurden in 1000 ml einer 10 gew.-%igen wässrigen $HNO_3$-Lösung für 7 h bei 70°C unter Rückfluß gerührt, der verbleibende Feststoff aus der resultierenden Aufschlämmung abfiltriert und mittels Wasser (25°C) nitratfrei gewaschen. Der erhaltene Filterkuchen wurde über Nacht unter Luft bei 110°C in einem Muffelofen getrocknet.
Die chemische Analyse des erhaltenen Feststoffs ergab die Zusammensetzung $Mo_1V_{0,29}Te_{0,13}Nb_{0,13}O_x$. Das zugehörige Röntgendiffraktogramm wies reine i-Phase aus.
Anschließend wurde das getrocknete Material wie in der DE-A 10119933 beschrieben in einer Retsch-Mühle gemahlen (Korngröße ≤ 0,12 mm) und wie in Beispiel A) a) der DE-A 10119933 zu einem Schalenkatalysator verarbeitet:
38 g gemahlene Aktivmasse; 150 g kugelförmige Trägerkörper mit einem Durchmesser von 2,2 bis 3,2 mm (Trägermaterial = Steatit C-220 der Fa. CeramTec, DE mit einer Oberflächenrauhigkeit Rz von 45 μm), Haftmittel = 30 ml eines Gemisches aus Glycerin und Wasser (Gewichtsverhältnis Glycerin : Wasser = 1 : 3), Trockendauer =16 h bei 150°C; der Aktivmassenanteil des resultierenden Schalenkatalysators lag bei 20 Gew.-% (bezogen auf das Gewicht des Schalenkatalysators).

2. Heterogen katalysierte partielle direkte Oxidation von Propan zu Acrylsäure bei verschiedenen Drucken

Mit dem Schalenkatalysator aus 1. wurde ein Reaktionsrohr (Länge 140 cm) aus V2A Stahl (Außendurchmesser = 60 mm, Innendurchmesser = 8,5 mm) beschickt. Die Beschickungslänge wurde zu 53 cm gewählt (= ca. 35,0 g des Schalenkatalysators). Eine Vorschüttung von 30 cm Länge aus den als Trägermaterial verwendeten Steatitkugeln diente der Positionierung der Katalysatorzone. Mit denselben Steatitkugeln wurde das Reaktionsrohr nach der Katalysatorzone abschließend aufgefüllt (Vorheizzone zur Anwärmung des Reaktionsgasausgangsgemisches). Das Reaktionsrohr wurde von außen auf seiner ganzen Länge mittels elektrischer Heizmatten beheizt. Die molare Zusammensetzung des Reaktionsgasausgangsgemischs betrug Propan : Luft: Wasser =1 : 15 : 14. Die nachfolgende Tabelle zeigt den resultierenden Propanumsatz bei einfachem Durchgang ($U^{PAN}$, mol-%) sowie die mit diesem Umsatz einhergehende Selektivität der Acrylsäurebildung ($S^{ACS}$, mol-%) in Abhängigkeit vom gewählten Eingangsdruck sowie der zugehörigen Temperatur der Heizmatten. Die Verweilzeit (bezogen auf das Katalysatorschüttungsvolumen) betrug in allen Fällen 2,4 s. Zusätzlich zeigt die Tabelle mit $S^{PEN}$ die Selektivität der Propennebenproduktbildung.

Tabelle

| Eingangsdruck [bar, absolut] | T [°C] | $U^{PAN}$ (mol-%) | $S^{ACS}$ (mol-%) | $S^{PEN}$ [mol.-%] |
|---|---|---|---|---|
| 1,3 | 390 | 28 | 70 | 10 |
| 1,3 | 440 | 53 | 39 | 5 |
| 6 | 330 | 32 | 69 | 5 |
| 6 | 350 | 50 | 70 | 3 |
| 6 | 390 | 77 | 60 | 1 |

Bei höherem Arbeitsdruck resultieren bei geringeren Temperaturen höhere Propanumsätze bei im wesentlichen unveränderter Selektivität der Zielproduktverbindung.

Im Übrigen enthielt das Produktgasgemisch in allen Fällen geringe Mengen weiterer Säuren wie z.B. Essigsäure sowie $CO_x$.

3. Isotherme Verdichterleistung beim erfindungsgemäßen Verfahren in Abhängigkeit vom Ausgangsdruck $P^3$ bei vorgegebenem Kreisgasverhältnis

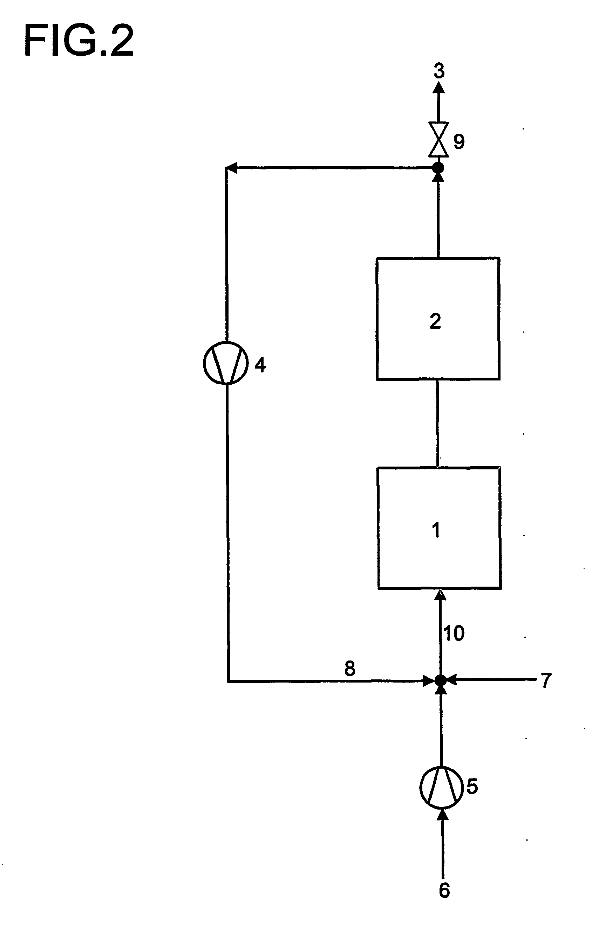

Das Verfahren gemäß 2. wird in einer apparativen Anordnung gemäß Figur 2 durchgeführt. Es wird ein Umsatz von 40 mol-% und eine Selektivität der Acrylsäurebildung von 70 mol-% zugrunde gelegt. Die molare Zusammensetzung Propan : Sauerstoff : Wasser entspricht der Zusammensetzung in 2.. Die Kreisgasführung beeinflusst den Stickstoffgehalt. Dabei haben die verschiedenen Ziffern die nachfolgende Bedeutung:

1 = Reaktionsstufe
2 = Aufarbeitungsstufe
3 = ausgelassener Teil des Restproduktgasgemisches
4 = Kreisgasverdichter (Gebläse)
5 = Luftverdichter
6 = Luft als Sauerstoffquelle
7 = frisches Propan und frischer Wasserdampf
8 = rückverdichtetes Kreisgas
9 = Drosselvorrichtung zur Regulierung des Ausgangsdrucks $P^3$
10 = Reaktionsgasausgangsgemisch mit Eingangsdruck $P^1$.

Das frische Propan und der frische Wasserdampf stehen im jeweils erforderlichen Eingangsdruck zur Verfügung.

D.h., eine Änderung von $P^3$ wirkt sich nur auf die Leistung des Kreisgasverdichters und auf die Leistung des Luftverdichters aus.

Der Einfachheit halber wird für beide Verdichter eine isotherme Verdichtung angenommen.

Gemäß VDI-Wärmeatlas, Verlag des Vereins Deutscher Ingenieure, Düsseldorf, 5. Auflage, 1988, Blatt La 1, beträgt die isotherme Verdichterleistung des Luftverdichters ($V^L$):

$$V^L = \frac{\dot{m}_L}{n_L} \cdot Z_L \cdot R \cdot T_L \cdot \ln\left(\frac{P^1}{1}\right),$$

mit

$\dot{m}_L$ =     Frischluftmassenstrom;
$n_L$ =     Wirkungsgrad des Luftverdichters;
$Z_L$ =     Realgasfaktor für Luft;
$R$ =     spezielle Gaskonstante = ideale Gaskonstante geteilt durch die Molmasse;
$T_L$ =     Temperatur, mit der die Frischluft aus der Umgebung angesaugt wird;

I = Normaldruck (Umgebungsdruck) = 1 bar mit dem die Luft angesaugt wird;

$P^1$ = Eingangsdruck in die Reaktionsstufe, auf den die Luft verdichtet wird;

D.h., $V^L = \dot{m}_L \cdot A \cdot \ln \cdot P^1$, wobei A eine Konstante ist;

In entsprechender Weise beträgt die isotherme Verdichterleistung des Kreisgasverdichters $V^K$:

$$V^K = \frac{\dot{m}_K}{n_K} \cdot Z_K \cdot R \cdot T_K \cdot \ln \frac{P^1}{P^3}.$$

D.h., $V^K = \dot{m}_K \cdot A' \cdot \ln \frac{P^1}{P^3}$, wobei A' eine Konstante ist, für die gilt: A' $\approx$ A.

Für die insgesamt aufzuwendende Verdichterleistung $V_{ges} = V^L + V^K$ gilt damit:

$$V_{ges} = \dot{m}_L \cdot A \cdot \ln \cdot P^1 + \dot{m}_K \cdot A' \ln \frac{P^1}{P^3}.$$

Mit $P^1 - P^3 = C \cdot \frac{1}{P^1}$, wobei C eine für die verwendete Reaktions- und Aufarbeitungsvorrichtung charakteristische Konstante ist, und

$$\frac{\dot{m}_K \cdot A'}{\dot{m}_L \cdot A} \approx \frac{\dot{m}_{\dot{K}}}{\dot{m}_L} = K_r = \text{Kreisgasverhältnis,}$$

ergibt sich:

$$V_{ges} = \dot{m}_K \cdot A \cdot \left[ \left(1 + \frac{1}{K_r}\right) \ln \left( \frac{P^3}{2} + \sqrt{\left(\frac{P^3}{2}\right)^2 + C} \right) - \ln P^3 \right].$$

Wählt man das Kreisgasverhältnis einmal zu 2,5 und einmal zu 3,5, und bestimmt man die Konstante C als C = (1,5 bar + $\Delta P_{1,5}$) · $\Delta P_{1,5}$,

wobei $\Delta P_{1,5}$ der bei einem Ausgangsdruck (Ausgang Aufarbeitungsstufe) von 1,5 bar über die Reaktionsstufe und die Aufarbeitungsstufe insgesamt erlittene Druckverlust ist, bei einem für das erfindungsgemäße Verfahren repräsentativen Druckverlust von 2 bar zu C = (1,5 bar + 2 bar) · 2 bar = 7 bar², so ergeben sich (die $\dot{m}_K$ sind so gewählt, dass die Raum-Zeit-Ausbeute an Acrylsäure in beiden Fällen gleich ist) bei $K_r$ = 2,5 mit dem Kreisgasmassenstrom $\dot{m}_K$ =166 kg/sec und bei $K_r$ = 3,5 mit dem Kreisgasmassenstrom $\dot{m}_K$ = 213 kg/sec bei den beiden gewählten Kreisgasverhältnissen die in Figur 3 gezeigten Verläufe von $V_{ges}$ in Abhängigkeit von $P^3$ (unter der gemäß der Ergebnisse in 2. konservativen Annahme von mit zunehmendem Druck gleich bleibendem Umsatz und Selektivität).

Ausgehend von $P^3$ = 1,5 bar nimmt mit zunehmendem $P^3$ die insgesamt aufzuwendende Verdichterleistung in beiden Fällen ab, was die Vorteilhaftigkeit der erfindungsgemäßen Verfahrensweise ausweist.

**Patentansprüche**

1. Verfahren der heterogen katalysierten partiellen direkten Oxidation von Propan und/oder iso-Butan zu wenigstens einem der Zielprodukte Acrylsäure, Methacrylsäure, bei dem man einer Reaktionsstufe, die, abgesehen von einem Eingang für das Reaktionsgasausgangsgemisch, gegebenenfalls weiteren Eingängen für Hilfsgase, und einem Ausgang für das Produktgasgemisch, gasseitig abgeschlossen ist, ein Propan und/oder iso-Butan, molekularen Sauerstoff und wenigstens ein inertes Verdünnungsgas enthaltendes Reaktionsgasausgangsgemisch mit einem Eingangsdruck $P^1$ zuführt, in der Reaktionsstufe durch Überleiten des Reaktionsgasausgangsgemischs bei erhöhter Temperatur über einen im festen Aggregatzustand befindlichen Katalysator das im Reaktionsausgangsgemisch enthaltene Propan und/oder iso-Butan direkt partiell zu wenigstens einem Zielprodukt oxidiert, und das Reaktionsgasgemisch als wenigstens ein Zielprodukt enthaltendes Produktgasgemisch mit dem Ausgangsdruck $P^2$ aus der Reaktionsstufe heraus und mit diesem Druck $P^2$ in eine Aufarbeitungsstufe führt, die gasseitig, abgesehen von einem Eingang für das Produktgasgemisch, gegebenenfalls weiteren Eingängen für Hilfsgase, und einem Ausgang für das Restproduktgasgemisch, abgeschlossen ist, in der Aufarbeitungsstufe aus dem Produktgasgemisch der Reaktionsstufe darin enthaltenes Zielprodukt in eine Flüssigphase grundabtrennt und das dabei verbleibende, nicht nur Propan und/oder iso-Butan sowie gegebenenfalls Propen und/oder iso-Buten enthaltende, Restproduktgasgemisch mit dem Ausgangsdruck $P^3$, für den $P^3 < P^1$ gilt, aus der Aufarbeitungsstufe herausführt und im Restproduktgasgemisch enthaltenes Propan und/oder iso-Butan in die Reaktionsstufe zurückführt, **dadurch gekennzeichnet, dass** $P^1$ so gewählt wird, dass $P^3 \geq 1{,}5$ bar beträgt und man das Restproduktgasgemisch in zwei Teilmengen gleicher Zusammensetzung aufteilt und davon die eine Teilmenge als Auslass ausschleust und die andere Teilmenge als Kreisgas rückführt und als Bestandteil des Reaktionsgasausgangsgemischs auf den Eingangsdruck $P^1$ verdichtet wieder der Reaktionsstufe zuführt.

2. Verfahren nach Anspruch 1, **dadurch gekennzeichnet, dass** das Restproduktgasgemisch wenigstens 5 Vol.-% an von Propan und/oder iso-Butan sowie von Propen und/oder iso-Buten verschiedenen Bestandteilen enthält.

3. Verfahren nach Anspruch 1, **dadurch gekennzeichnet, dass** das Restproduktgasgemisch wenigstens 10 Vol.-% an von Propan und/oder iso-Butan sowie von Propen und/oder iso-Buten verschiedenen Bestandteilen enthält.

4. Verfahren nach einem der Ansprüche 1 bis 3, **dadurch gekennzeichnet, dass** der Druck $P^3 \geq 1{,}5$ und $\leq 25$ bar beträgt.

5. Verfahren nach einem der Ansprüche 1 bis 3, **dadurch gekennzeichnet, dass** der Druck $P^3 \geq 1{,}5$ und $\leq 20$ bar beträgt.

6. Verfahren nach einem der Ansprüche 1 bis 3, **dadurch gekennzeichnet, dass** der Druck $P^3 \geq 1{,}5$ und $\leq 10$ bar beträgt.

7. Verfahren nach einem der Ansprüche 1 bis 3, **dadurch gekennzeichnet, dass** der Druck $P^3 \geq 2$ bar und $\leq 8$ bar beträgt.

8. Verfahren nach einem der Ansprüche 1 bis 7, **dadurch gekennzeichnet, dass** der Druck $P^1$ 1 bis 4 bar oberhalb des Druckes $P^3$ liegt.

9. Verfahren nach einem der Ansprüche 1 bis 7, **dadurch gekennzeichnet, dass** der Druck $P^1$ 1,5 bis 3,5 bar oberhalb des Druckes $P^3$ liegt.

10. Verfahren nach einem der Ansprüche 1 bis 9, **dadurch gekennzeichnet, dass** $P^1$ 3 bis 10 bar beträgt.

11. Verfahren nach einem der Ansprüche 1 bis 9, **dadurch gekennzeichnet, dass** $P^1$ 4 bis 8 bar beträgt.

12. Verfahren nach einem der Ansprüche 1 bis 11, **dadurch gekennzeichnet, dass** die als Auslass ausgeschleuste Teilmenge des Restproduktgasgemisches über einen Expander ausgeschleust wird.

13. Verfahren nach einem der Ansprüche 1 bis 12, **dadurch gekennzeichnet, dass** die Reaktionsstufe ein mit Katalysator beschickter Rohrbündelreaktor oder Wirbelbettreaktor ist.

14. Verfahren nach einem der Ansprüche 1 bis 13, **dadurch gekennzeichnet, dass** die Aufarbeitungsstufe eine Absorptionskolonne oder eine Kolonne zur fraktionierenden Kondensation oder eine Hintereinanderschaltung von Quenchstufen ist.

15. Verfahren nach einem der Ansprüche 1 bis 14, **dadurch gekennzeichnet, dass** der Katalysator als Aktivmasse eine Multimetalloxidmasse aufweist, die die Elemente Mo, V, wenigstens eines der beiden Elemente Te und Sb und wenigstens eines der Elemente aus der Gruppe umfassend Nb, Ta, W, Ti, Al, Zr, Cr, Mn, Ga, Fe, Ru, Co, Cs, Ca, Sr, Ba, Rh, Ni, Pd, Pt, La, Pb, Cu, Re, Ir, Y, Pr, Nd, Tb, Bi, B, Ce, Sn Zn, Si, Na, Li, K, Mg, Ag, Au und In in Kombination enthält.

16. Verfahren nach einem der Ansprüche 1 bis 14, **dadurch gekennzeichnet, dass** der Katalysator als Aktivmasse eine Multimetalloxidmasse aufweist, die die Elementkombination mit der Stöchiometrie I

$$Mo_1V_bM^1_cM^2_d \qquad (I),$$

mit

M$^1$ = Te und/oder Sb,
M$^2$ = wenigstens eines der Elemente aus der Gruppe umfassend Nb, Ta, W, Ti, Al, Zr, Cs, Ca, Sr, Ba, Cr, Mn, Ga, Fe, Ru, Co, Rh, Ni, Pd, Pt, La, Bi, Pb, Cu, Re, Ir, Y, Pr, Nd, Tb, Ce, Sn, Zn, Si, Na, Li, K, Mg, Ag, Au und In,
b = 0,01 bis 1
c = > 0 bis 1 und
d = > 0 bis 1

enthält.

17. Verfahren nach einem der Ansprüche 1 bis 16, **dadurch gekennzeichnet, dass** als Sauerstoffquelle Luft verwendet wird.

18. Verfahren nach einem der Ansprüche 1 bis 17, **dadurch gekennzeichnet, dass** die Reaktionstemperatur 200 bis 700°C beträgt.

19. Verfahren nach einem der Ansprüche 1 bis 18, **dadurch gekennzeichnet, dass** das Reaktionsgasausgangsgemisch
0,5 bis 15 Vol.-% Propan oder iso-Butan,
10 bis 90 Vol.-% Luft,
0 bis 50 Vol.-% Wasserdampf und
als Restmenge Kreisgas
enthält.

20. Verfahren nach einem der Ansprüche 1 bis 18, **dadurch gekennzeichnet, dass** das Reaktionsgasausgangsgemisch
0,5 bis 15 Vol.-% Propan oder iso-Butan,
10 bis 90 Vol.-% Luft,
10 bis 50 Vol.-% Wasserdampf und
als Restmenge Kreisgas
enthält.

21. Verfahren nach einem der Ansprüche 1 bis 18, **dadurch gekennzeichnet, dass** das Reaktionsgasausgangsgemisch
70 bis 90 Vol.-% Propan oder iso-Butan,
5 bis 25 Vol.-% molekularer Sauerstoff,
0 bis 25 Vol.-% Wasserdampf und
als Restmenge Kreisgas
enthält.

**22.** Verfahren nach einem der Ansprüche 1 bis 21, **dadurch gekennzeichnet, dass** der Umsatz aus Propan und/oder iso-Butan, bezogen auf einmaligen Durchgang des Reaktionsgasgemisches durch die Reaktionsstufe, 10 bis 70 mol-% beträgt.

**23.** Verfahren nach Anspruch 22, **dadurch gekennzeichnet, dass** die Selektivität der Zielproduktbildung 40 bis 98 mol-% beträgt.

**24.** Verfahren nach einem der Ansprüche 1 bis 23, **dadurch gekennzeichnet, dass** die Grundabtrennung von im Produktgasgemisch der Reaktionsstufe enthaltenem Zielprodukt in die Flüssigphase so erfolgt, dass im verbleibenden Restproduktgasgemisch das molare Verhältnis W des darin enthaltenen Wasserdampf zum darin enthaltenen Propan höchstens 50 % kleiner ist, als das entsprechende molare Verhältnis W' im Produktgasgemisch der Reaktionsstufe.

**25.** Verfahren nach einem der Ansprüche 1 bis 24, **dadurch gekennzeichnet, dass** die Grundabtrennung von im Produktgasgemisch der Reaktionsstufe enthaltenem Zielprodukt in die Flüssigphase in einer Absorptionskolonne durch Absorption in ein organisches Lösemittel so erfolgt, dass der Ablauf aus der Absorptionskolonne einphasig ist.

**26.** Verfahren nach einem der Ansprüche 1 bis 25, **dadurch gekennzeichnet, dass** man aus der als Auslass ausgeschleusten Teilmenge des Restproduktgasgemisches das darin enthaltene Propan und/oder iso-Butan sowie gegebenenfalls Propen und/oder iso-Buten abtrennt und auf den Eingangsdruck $P^1$ rückverdichtet in die Reaktionsstufe rückführt.

**27.** Verfahren nach einem der Ansprüche 1 bis 26, **dadurch gekennzeichnet, dass** das Mengenverhältnis V von derjenigen Teilmenge des Restproduktgasgemisches, die als Kreisgas rückgeführt wird, zu derjenigen Teilmenge des Restproduktgasgemisches, die als Auslass ausgeschleust wird, $\geq 0,5$ und $\leq 30$ beträgt.

**28.** Verfahren nach einem der Ansprüche 1 bis 26, **dadurch gekennzeichnet, dass** das Mengenverhältnis V von derjenigen Teilmenge des Restproduktgasgemisches, die als Kreisgas rückgeführt wird, zu derjenigen Teilmenge des Restproduktgasgemisches, die als Auslass ausgeschleust wird, $\geq 2$ und $\leq 25$ beträgt.

**29.** Verfahren nach einem der Ansprüche 1 bis 26, **dadurch gekennzeichnet, dass** das Mengenverhältnis V von derjenigen Teilmenge des Restproduktgasgemisches, die als Kreisgas rückgeführt wird, zu derjenigen Teilmenge des Restproduktgasgemisches, die als Auslass ausgeschleust wird, $\geq 3$ und $\leq 20$ beträgt.

**30.** Verfahren nach einem der Ansprüche 1 bis 29, **dadurch gekennzeichnet, dass** das Kreisgas mit einem Gebläse auf den Eingangsdruck $P^1$ rückverdichtet wird.

**31.** Verfahren nach einem der Ansprüche 1 bis 30, **dadurch gekennzeichnet, dass** als Sauerstoffquelle Luft verwendet wird, die mittels eines Radialverdichters auf den Eingangsdruck $P^1$ verdichtet wird.

**32.** Verfahren nach einem der Ansprüche 1 bis 31, **dadurch gekennzeichnet, dass** es ein Verfahren der partiellen direkten Oxidation von Propan zu Acrylsäure ist.

**Claims**

**1.** A process for heterogeneously catalyzed partial direct oxidation of propane and/or isobutane to at least one of the target products acrylic acid, methacrylic acid, by feeding a starting reaction gas mixture comprising propane and/or isobutane, molecular oxygen and at least one inert diluent gas and having a inlet pressure $P^1$ to a reaction stage which, apart from an inlet for the starting reaction gas mixture, if appropriate further inlets for auxiliary gases, and an outlet for the product gas mixture, is sealed on the gas side, in the reaction stage directly oxidizing the propane and/or isobutane present in the starting reaction gas mixture partially to at least one target product by passing the starting reaction gas mixture at elevated temperature over a solid state catalyst, and conducting the reaction gas mixture as a product gas mixture comprising at least one target product and having the outlet pressure $P^2$ out of the reaction stage and, with this pressure $P^2$, into a workup stage which, apart from an inlet for the product gas mixture, if appropriate further inlets for auxiliary gases, and an outlet for the residual product gas mixture, is sealed on the gas side, in the workup stage basically separating target product present in the product gas mixture of the reaction stage from said product gas mixture into a liquid phase and conducting the remaining residual product gas mixture which comprises not only propane and/or isobutane but possibly also propene and/or isobutene and has the outlet pressure $P^3$, where $P^3 < P^1$, out of the workup stage and recycling propane and/or isobutane present in the residual product gas mixture into the reaction stage, which comprises selecting $P^1$ in such a way that $P^3 \geq 1.5$ bar and dividing the residual product gas mixture into two portions of the same composition and discharging one portion as output and recycling the other portion as cycle gas and feeding it back to the reaction stage, compressed to the inlet pressure $P^1$, as a constituent of the starting reaction gas mixture.

2. The process according to claim 1, wherein the residual product gas mixture comprises at least 5% by volume of constituents other than propane and/or isobutane and also other than propene and/or isobutene.

3. The process according to claim 1, wherein the residual product gas mixture comprises at least 10% by volume of constituents other than propane and/or isobutane and also other than propene and/or isobutene.

4. The process according to any of claims 1 to 3, wherein the pressure $P^3 \geq 1.5$ bar and $\leq 25$ bar.

5. The process according to any of claims 1 to 3, wherein the pressure $P^3 \geq 1.5$ bar and $\leq 20$ bar.

6. The process according to any of claims 1 to 3, wherein the pressure $P^3 \geq 1.5$ bar and $\leq 10$ bar.

7. The process according to any of claims 1 to 3, wherein the pressure $P^3 \geq 2$ bar and $\leq 8$ bar.

8. The process according to any of claims 1 to 7, wherein the pressure $P^1$ is from 1 to 4 bar above the pressure $P^3$.

9. The process according to any of claims 1 to 7, wherein the pressure $P^1$ is from 1.5 to 3.5 bar above the pressure $P^3$.

10. The process according to any of claims 1 to 9, wherein $P^1$ is from 3 to 10 bar.

11. The process according to any of claims 1 to 9, wherein $P^1$ is from 4 to 8 bar.

12. The process according to any of claims 1 to 11, wherein the portion of the residual product gas mixture which is discharged as output is discharged via an expander.

13. The process according to any of claims 1 to 12, wherein the reaction stage is a catalyst-charged tube bundle reactor or fluidized bed reactor.

14. The process according to any of claims 1 to 13, wherein the workup stage is an absorption column or a column for fractional condensation or a series arrangement of quench stages.

15. The process according to any of claims 1 to 14, wherein the active composition of the catalyst is a multimetal oxide composition which comprises the elements Mo, V, at least one of the two elements Te and Sb, and at least one of the elements from the group comprising Nb, Ta, W, Ti, Al, Zr, Cr, Mn, Ga, Fe, Ru, Co, Cs, Ca, Sr, Ba, Rh, Ni, Pd, Pt, La, Pb, Cu, Re, Ir, Y, Pr, Nd, Tb, Bi, B, Ce, Sn, Zn, Si, Na, Li, K, Mg, Ag, Au and In in combination.

16. The process according to any of claims 1 to 14, wherein the active composition of the catalyst is a multimetal oxide composition which contains the element combination having the stoichiometry I

$$Mo_1V_bM^1_cM^2_d \qquad (I),$$

where

$M^1$ = Te and/or Sb,
$M^2$ = at least one of the elements from the group comprising Nb, Ta, W, Ti, Al, Zr, Cs, Ca, Sr, Ba, Cr, Mn, Ga, Fe, Ru, Co, Rh, Ni, Pd, Pt, La, Bi, Pb, Cu, Re, Ir, Y, Pr, Nd, Tb, Ce, Sn, Zn, Si, Na, Li, K, Mg, Ag, Au and In,
$b$ = from 0.01 to 1
$c$ = from > 0 to 1 and
$d$ = from > 0 to 1.

17. The process according to any of claims 1 to 16, wherein the oxygen source used is air.

18. The process according to any of claims 1 to 17, wherein the reaction temperature is from 200 to 700°C.

19. The process according to any of claims 1 to 18, wherein the starting reaction gas mixture comprises
from 0.5 to 15% by volume of propane or isobutane,
from 10 to 90% by volume of air,
from 0 to 50% by volume of steam and
a remainder of cycle gas.

20. The process according to any of claims 1 to 18, wherein the starting reaction gas mixture comprises
from 0.5 to 15% by volume of propane or isobutane,
from 10 to 90% by volume of air,
from 10 to 50% by volume of steam and
a remainder of cycle gas.

21. The process according to any of claims 1 to 18, wherein the starting reaction gas mixture comprises
from 70 to 90% by volume of propane or isobutane,
from 5 to 25% by volume of molecular oxygen,
from 0 to 25% by volume of steam and
a remainder of cycle gas.

22. The process according to any of claims 1 to 21, wherein the conversion from propane and/or isobutane, based on single pass of the reaction gas mixture through the reaction stage, is from 10 to 70 mol%.

23. The process according to claim 22, wherein the selectivity of the target product formation is from 40 to 98 mol%.

24. The process according to any of claims 1 to 23, wherein the target product present in the product gas mixture of the reaction stage is basically separated into the liquid phase in such a way that the molar ratio W of the steam present in the remaining residual product gas mixture to the propane present therein is at least 50% smaller than the corresponding molar ratio W' in the product gas mixture of the reaction stage.

25. The process according to any of claims 1 to 24, wherein the target product present in the product gas mixture of the reaction stage is basically separated into the liquid phase in an absorption column by absorption into an organic solvent in such a way that the discharge from the absorption column is monophasic.

26. The process according to any of claims 1 to 25, wherein the propane and/or isobutane and also any propene and/or isobutene present in the portion of the residual product gas mixture which is discharged as output are removed from said residual product gas mixture and recycled into the reaction stage, recompressed to the inlet pressure $P^1$.

27. The process according to any of claims 1 to 26, wherein the ratio V of that portion of the residual product gas mixture which is recycled as cycle gas to that portion of the residual product gas mixture which is discharged as output is $\geq 0.5$ and $\leq 30$.

28. The process according to any of claims 1 to 26, wherein the ratio V of that portion of the residual product gas mixture which is recycled as cycle gas to that portion of the residual product gas mixture which is discharged as output is $\geq 2$ and $\leq 25$.

29. The process according to any of claims 1 to 26, wherein the ratio V of that portion of the residual product gas mixture which is recycled as cycle gas to that portion of the residual product gas mixture which is discharged as output is $\geq 3$ and $\leq 20$.

30. The process according to any of claims 1 to 29, wherein the cycle gas is recompressed to the inlet pressure $P^1$ using a blower.

31. The process according to any of claims 1 to 30, wherein the oxygen source used is air which is compressed to the inlet pressure $P^1$ by means of a radial compressor.

32. The process according to any of claims 1 to 31, which is a process for partial direct oxidation of propane to acrylic acid.

**Revendications**

1. Procédé d'oxydation directe partielle à catalyse hétérogène de propane et/ou d'isobutane en au moins l'un des produits cibles que sont l'acide acrylique, l'acide méthacrylique, dans lequel on amène à un étage de réaction qui, abstraction faite d'une entrée pour le mélange réactionnel gazeux de départ, le cas échéant d'autres entrées de gaz adjuvants, et d'une sortie du mélange gazeux de produits, est obturé du côté gaz, un mélange réactionnel gazeux de départ contenant du propane et/ou de l'isobutane, de l'oxygène moléculaire et au moins un gaz de dilution inerte, avec une pression d'entrée $P^1$, et dans l'étage de réaction, par passage du mélange réactionnel gazeux de départ à température élevée sur un catalyseur se trouvant à l'état d'agrégat solide, le propane et/ou l'isobutane contenus dans le mélange réactionnel gazeux de départ sont directement partiellement oxydés en au moins un produit cible, et le mélange réactionnel gazeux est évacué de l'étage de réaction, en tant que mélange gazeux de produits contenant au moins un produit cible, et à une pression de sortie $P^2$, et envoyé à cette pression $P^2$ à un étage de retraitement, qui est obturé du côté gaz, à l'exception d'une entrée pour le mélange gazeux de produits, le cas échéant d'autres entrées pour des gaz adjuvants et d'une sortie pour le mélange gazeux de produits résiduel, dans l'étage de retraitement on sépare essentiellement du mélange gazeux de produits de l'étage de réaction le produit cible qu'il contient, dans une phase liquide, et le mélange résiduel de produits gazeux ainsi restant contenant non seulement du propane et/ou de l'isobutane ainsi que le cas échéant du propène et/ou de l'isobutène est évacué de l'étage de retraitement avec une pression de sortie $P^3$, avec $P^3 < P^1$, et le propane et/ou l'isobutane contenus dans le mélange gazeux de produits résiduel sont renvoyés dans l'étage de réaction, **caractérisé en ce que** $P^1$ est choisie de manière à ce que $P^3 \geq 1,5$ bar, et le mélange résiduel de produits gazeux est séparé en deux quantités partielles de même composition, une quantité partielle étant éclusée en tant qu'effluent et l'autre quantité partielle étant renvoyée en tant que gaz de circulation et que gaz de circulation et étant ramenée à l'étage de réaction, comprimée à la pression d'entrée $P^1$, en tant que constituant du mélange réactionnel gazeux de départ.

2. Procédé suivant la revendication 1, **caractérisé en ce que** le mélange résiduel de produits gazeux contient au moins 5 % en volume de constituants différents du propane et/ou de l'isobutane ainsi que du propène et/ou de l'isobutène.

3. Procédé suivant la revendication 1, **caractérisé en ce que** le mélange résiduel de produits gazeux contient au moins 10 % en volume de constituants différents du propane et/ou de l'isobutane et du propène et/ou de l'isobutène.

4. Procédé suivant l'une quelconque des revendications 1 à 3, **caractérisé en ce que** la pression $P^3$ est supérieure ou égale à 1,5 bar et inférieure ou égale à 25 bars.

5. Procédé suivant l'une quelconque des revendications 1 à 3, **caractérisé en ce que** la pression $P^3$ est supérieure ou égale à 1,5 bar et inférieure ou égale à 20 bars.

6. Procédé suivant l'une quelconque des revendications 1 à 3, **caractérisé en ce que** la pression $P^3$ est supérieure ou égale à 1,5 bar et inférieure ou égale à 10 bars.

7. Procédé suivant l'une quelconque des revendications 1 à 3, **caractérisé en ce que** la pression $P^3$ est supérieure ou égale à 2 bars et inférieure ou égale à 8 bars.

8. Procédé suivant l'une quelconque des revendications 1 à 7, **caractérisé en ce que** la pression $P^1$ est supérieure de 1 à 4 bars à la pression $P^3$.

9. Procédé suivant l'une quelconque des revendications 1 à 7, **caractérisé en ce que** la pression $P^1$ est supérieure de 1,5 à 3,5 bars à la pression $P^3$.

10. Procédé suivant l'une quelconque des revendications 1 à 9, **caractérisé en ce que** $P^1$ vaut de 3 à 10 bars.

11. Procédé suivant l'une quelconque des revendications 1 à 9, **caractérisé en ce que** $P^1$ vaut de 4 à 8 bars.

12. Procédé suivant l'une quelconque des revendications 1 à 11, **caractérisé en ce que** la quantité partielle éclusée du mélange gazeux de produits résiduel en tant qu'effluent est éclusée via un expanseur.

13. Procédé suivant l'une quelconque des revendications 1 à 12, **caractérisé en ce que** l'étage de réaction est un réacteur à faisceau de tubes ou un réacteur à lit fluidisé muni d'un catalyseur.

**14.** Procédé suivant l'une quelconque des revendications 1 à 13, **caractérisé en ce que** l'étage de retraitement est une colonne d'absorption ou une colonne de condensation fractionnée ou une installation en série d'étages de refroidissement rapide.

**15.** Procédé suivant l'une quelconque des revendications 1 à 14, **caractérisé en ce que** le catalyseur présente en tant que masse active une masse d'oxydes multimétalliques contenant les éléments Mo, V, au moins l'un des deux éléments Te et Sb et au moins l'un des éléments du groupe comprenant Nb, Ta, W, Ti, Al, Zr, Cr, Mn, Ga, Fe, Ru, Co, Cs, Ca, Sr, Ba, Rh, Ni, Pd, Pt, La, Pb, Cu, Re, Ir, Y, Pr, Nd, Tb, Bi, B, Ce, Sn, Zn, Si, Na, Li, K, Mg, Ag, Au et In en combinaison.

**16.** Procédé suivant l'une quelconque des revendications 1 à 14, **caractérisé en ce que** le catalyseur présente en tant que masse active une masse d'oxydes multimétalliques contenant la combinaison d'éléments avec la stoechiométrie I :

$$Mo_1V_bM^1_cM^2_d \qquad (I),$$

avec

M$^1$ = Te et/ou Sb,
M$^2$ = au moins l'un des éléments du groupe comprenant Nb, Ta, W, Ti, Al, Zr, Cs, Ca, Sr, Ba, Cr, Mn, Ga, Fe, Ru, Co, Rh, Ni, Pd, Pt, La, Bi, Pb, Cu, Re, Ir, Y, Pr, Nd, Tb, Ce, Sn, Zn, Si, Na, Li, K, Mg, Ag, Au et In,
b = 0,01 à 1
c = >0 à 1 et
d = >0 à 1.

**17.** Procédé suivant l'une quelconque des revendications 1 à 16, **caractérisé en ce que** l'on utilise de l'air en tant que source d'oxygène.

**18.** Procédé suivant l'une quelconque des revendications 1 à 17, **caractérisé en ce que** la température de réaction est de 200 à 700°C.

**19.** Procédé suivant l'une quelconque des revendications 1 à 18, **caractérisé en ce que** le mélange réactionnel gazeux de départ contient :

de 0,5 à 15 % en volume de propane ou d'isobutane,
de 10 à 90 % en volume d'air,
de 0 à 50 % en volume de vapeur d'eau et
le reste étant du gaz de circulation.

**20.** Procédé suivant l'une quelconque des revendications 1 à 18, **caractérisé en ce que** le mélange réactionnel gazeux de départ contient :

de 0,5 à 15 % en volume de propane ou d'isobutane,
de 10 à 90 % en volume d'air,
de 10 à 50 % en volume de vapeur d'eau et
le reste étant du gaz de circulation.

**21.** Procédé suivant l'une quelconque des revendications 1 à 18, **caractérisé en ce que** le mélange réactionnel gazeux de départ contient :

de 70 à 90 % en volume de propane ou d'isobutane,
de 5 à 25 % en volume d'oxygène moléculaire,
de 0 à 25 % en volume de vapeur d'eau et
le reste étant du gaz de circulation.

**22.** Procédé suivant l'une quelconque des revendications 1 à 21, **caractérisé en ce que** la conversion de propane et/ou d'isobutane, sur base d'un passage du mélange réactionnel gazeux dans l'étage de réaction, est de 10 à 70 % molaires.

**23.** Procédé suivant la revendication 22, **caractérisé en ce que** la sélectivité pour la formation du produit cible est de 40 à 98 % molaires.

**24.** Procédé suivant l'une quelconque des revendications 1 à 23, **caractérisé en ce que** la séparation de base du produit cible contenu dans le mélange de produits gazeux de l'étage de réaction se fait, dans la phase liquide, de manière à ce que, dans le mélange résiduel de produits gazeux, la proportion molaire W de la vapeur d'eau contenue au propane contenu soit d'au maximum de 50 % inférieure à la proportion molaire correspondante W' dans le mélange gazeux de produits de l'étage de réaction.

**25.** Procédé suivant l'une quelconque des revendications 1 à 24, **caractérisé en ce que** la séparation de base du produit cible contenu dans le mélange gazeux de produits de l'étage de réaction, dans la phase liquide, se fait dans une colonne d'absorption par absorption dans un solvant organique, de manière à ce que la sortie de la colonne d'absorption soit à une phase.

**26.** Procédé suivant l'une quelconque des revendications 1 à 25, **caractérisé en ce que** l'on sépare de la quantité partielle éclusée en tant qu'effluent du mélange résiduel de produits gazeux le propane et/ou l'isobutane qu'elle contient et le cas échéant le propène et/ou l'isobutène, qu'on les recomprime à la pression d'entrée $P^1$ et les renvoie dans l'étage de réaction.

**27.** Procédé suivant l'une quelconque des revendications 1 à 26, **caractérisé en ce que** la proportion quantitative V de la quantité partielle de mélange résiduel de produits gazeux recyclée en tant que gaz de circulation à la quantité partielle de mélange résiduel de produits gazeux éclusée en tant qu'effluent est $\geq 0,5$ et $\leq 30$.

**28.** Procédé suivant l'une quelconque des revendications 1 à 26, **caractérisé en ce que** la proportion quantitative V de la quantité partielle de mélange résiduel de produits gazeux recyclée en tant que gaz de circulation à la quantité partielle de mélange résiduel de produits gazeux éclusée en tant qu'effluent est $\geq 2$ et $\leq 25$.

**29.** Procédé suivant l'une quelconque des revendications 1 à 26, **caractérisé en ce que** la proportion quantitative V de la quantité partielle de mélange résiduel de produits gazeux recyclée en tant que gaz de circulation à la quantité partielle de mélange résiduel de produits gazeux éclusée en tant qu'effluent est $\geq 3$ et $\leq 20$.

**30.** Procédé suivant l'une quelconque des revendications 1 à 29, **caractérisé en ce que** le gaz de circulation est recomprimé par une soufflante à la pression d'entrée $P^1$.

**31.** Procédé suivant l'une quelconque des revendications 1 à 30, **caractérisé en ce que** l'on utilise comme source d'oxygène de l'air, qui est comprimé par un compresseur radial à la pression d'entrée $P^1$.

**32.** Procédé suivant l'une quelconque des revendications 1 à 31, **caractérisé en ce qu'**il constitue un procédé d'oxydation partielle directe de propane en acide acrylique.

# FIG.1

# FIG.2

# FIG.3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 529853 A
- EP 603836 A
- EP 608838 A
- EP 895809 A
- DE 19835247 A
- DE 10051419 A
- DE 10122027 A
- EP 1254707 A
- EP 1254709 A
- EP 1192987 A
- EP 1090684 A
- DE 10254279 A
- DE 19924532 A
- DE 10261186 A
- DE 10119933 A
- EP 1193240 A
- EP 495504 A
- DE 10259023 A
- WO 02081421 A
- JP 3170445 A
- EP 609122 A
- EP 747349 A
- JP 7232071 A
- JP 11169716 A
- JP 10057813 A
- JP 2000037623 A
- JP 10036311 A
- WO 0029105 A
- EP 767164 A
- DE 10029338 A
- JP 8057319 A
- JP 10028862 A
- JP 11043314 A
- JP 11574719 A
- WO 0029106 A
- JP 10330343 A
- JP 11285637 A
- JP 310539 A
- JP 11042434 A
- JP 11343261 A
- JP 3423262 A
- WO 9903825 A
- JP 7053448 A
- JP 2000051693 A
- JP 11263745 A
- DE 10046672 A
- DE 10118814 A
- JP 2000143244 A
- EP 318295 A
- DE 19832033 A
- DE 19836359 A
- EP 962253 A
- DE 10033121 A
- DE 10145958 A
- DE 10303526 A
- DE 10254278 A
- EP 700714 A
- EP 700893 A
- DE 19927624 A
- DE 19948242 A
- DE 19948241 A
- DE 19910508 A
- DE 19910506 A
- WO 020811421 A
- JP 2001026269 A
- EP 990636 A
- JP 2000327651 A
- EP 925272 A
- EP 695736 A
- EP 778255 A
- DE 2136396 A
- DE 2449780 A
- DE 4308087 A
- EP A A
- EP 982287 A
- EP 982289 A
- EP 982288 A
- DE 19631645 A
- DE 19606877 A
- DE 19838845 A
- DE 10200583 A
- DE 10053086 A
- DE 19627847 A
- DE 19740253 A
- DE 19740252 A
- DE 19814387 A
- DE 10247240 A
- WO 0196271 A
- DE 101199331 A